# EUROPÄISCHE PATENTSCHRIFT

(11) **EP 2 080 503 B1**
(45) Veröffentlichungstag und Bekanntmachung des Hinweises auf die Patenterteilung: **14.09.2011**
(21) Anmeldenummer: 08000956.6
(22) Anmeldetag: 18.01.2008
(51) Int. Cl.: A61K 6/083, A61K 6/093

(54) **Dentalmaterialien mit oberflächenfunktionalisierten Füllstoffen**
Dental materials with surface functional fillers
Matériaux dentaires dotés d'agents de remplissage à surface fonctionnalisée

(43) Veröffentlichungstag der Anmeldung: 22.07.2009
(73) Patentinhaber: Ivoclar Vivadent AG, 9494 Schaan (LI)
(72) Erfinder: Moszner, Norbert, Prof. Dr., 9493 Mauren (LI); Angermann, Jörg, Dr., 7320 Sargans (CH); Klapdohr, Simone, Dr., 83022 Rosenheim (DE); Rheinberger, Volker, Dr., 9490 Vaduz (LI)
(74) Vertreter: UEXKÜLL & STOLBERG

(56) Entgegenhaltungen:
- EP-A2- 1 900 426
- WO-A-03/070198
- WO-A1-84/00375
- FR-A- 2 889 704
- US-A1- 2003 175 217
- US-A1- 2006 247 329

## Beschreibung

Die vorliegende Erfindung betrifft Zusammensetzungen auf Basis von oberflächenfunktionalisierten Füllstoffen, die sich besonders als Dentalmaterialien eignen. Die Erfindung betrifft auch oberflächenfunktionalisierte Füllstoffe, ein Verfahren zur Herstellung der erfindungsgemäßen Zusammensetzungen und Füllstoffe, sowie deren Verwendung als Dentalmaterialien zur Herstellung von Adhäsiven, Beschichtungen oder Kompositen.

Zur Vermittlung der Haftung von Dentalmaterialien auf der Zahnhartsubstanz (Schmelz und Dentin) ist es bekannt, in den Dentalmaterialien polymerisationsfähige Monomere zu verwenden, die mit Hydroxylapatit oder Kollagen bindende Wechselwirkungen eingehen können, beispielsweise ethylenisch ungesättigte Monomere, die Aldehyd-, β-Diketon-, β-Ketoester-, Carbonsäureanhydrid- oder Säuregruppen enthalten (vgl. N. Moszner, U. Salz, J. Zimmermann, Dental Materials 21 (2005) 895-910; U. Salz, S. W. Shalaby, Polymers for Dental and Orthopedic Applications, CRC Press, Boca Raton etc. 2007, 69ff.). So werden Methacrylate, die Carbonsäure-, Carbonsäureanhydrid-, Phosphonsäure-, Phosphorsäure- oder Sulfonsäuregruppen enthalten, oder Glutaraldehyd als Komponenten in kommerziellen Schmelz-Dentin-Adhäsiven eingesetzt.

Darüber hinaus werden bestimmte Säuren auch als Reaktionskomponenten in dentalen Zementen eingesetzt, beispielsweise einfache Säuren wie Phosphorsäure als Reaktionspartner für ZnO in Phosphatzementen, Polyacrylsäure als Reaktionspartner für ZnO in Polycarboxylatzementen, Copolymere aus Acrylsäure und Itaconsäure als Reaktionspartner für Calciumaluminiumsilikatgläser in Glasionomerzementen oder auch bestimmte Säuremonomere als Reaktionskomponente für Calciumaluminiumsilikatgläser in Compomeren (vgl. E. C. Combe, F. J. T. Burke, W. H. Douglas, Dental Biomaterials, Kluwer Academic. Publishers, Boston etc. 1999, 211ff., 221ff., 233ff.; U. Salz, S. W. Shalaby, Polymers for Dental and Orthopedic Applications, CRC Press, Boca Raton etc. 2007, 49 ff).

WO 03/070198 beschreibt dentale Adhäsivzusammensetzungen mit Nanopartikeln, die durch Hydrolyse von Mischungen erhalten werden, die Organosiliziumverbindungen mit polymerisierbaren Gruppen und Organosiliziumverbindungen mit sauren Gruppen enthalten.

Zur mechanischen Verstärkung von Dentalmaterialien werden häufig Füllstoffe verwendet, insbesondere silikatische und nichtsilikatische anorganische Füllstoffe. Zu den hauptsächlich eingesetzten silikatischen Füllstoffen gehören gemahlene Gläser wie z.B. Barium-Silikatgläser (US 4,220,582), Strontium-Silikatgläser (DE 43 23 143), Lithium-Aluminium-Silikat-Gläser (GB 1 488 403) und röntgenopaque Aluminium-Fluoro-Silikat gläser, die vor allem in Methacrylat-verstärkten Glasionomeren Verwendung finden (US 5,367,002, US 5,871,360). Reine Siliziumoxid-Füllstoffe finden ebenfalls in Dentalmaterialien Anwendung (DE 24 05 578). Bekannt sind auch Mischoxide auf Basis von Silizium- und Zirkonoxid (DE 32 47 800). Neben der mechanischen Verstärkung werden Füllstoffe auch zur Erhöhung der Röntgenopazität sowie zur Einstellung von Konsistenz und Transparenz verwendet. Nichtsilikatische Füllstoffe werden insbesondere als Röntgenkontrastmittel eingesetzt, beispielweise Zirkonoxid (WO 00/69392), Tantaloxid (WO 98/13008) oder Yttriumoxid (DE 100 18 405). Aluminium- und Titanoxid dienen wegen ihres hohen Brechungsindex als Trübungsmittel.

Durch Modifizierung ihrer Oberfläche können verschiedene Eigenschaften von Füllstoffen eingestellt werden. Im Falle von anorganischen, silikatischen Füllstoffen kann hierzu beispielsweise eine Silanisierung durchgeführt werden. So ist zur Einstellung von hydrophilen bzw. hydrophoben Eigenschaften, UV-Absorptionsvermögen und schmutzabweisenden Eigenschaften von Füllstoffen sowie zur Verbesserung ihrer Suspendierbarkeit und Einbindungsfähigkeit in eine Kunststoffmatrix aus der DE 10 2004 022 566 A1 ein Verfahren zur Beschichtung von Glas-, Glaskeramik- und/oder Keramikpulvern bekannt, in dem mit bestimmten funktionellen Gruppen versehene Silane als Beschichtungsreagenzien verwendet werden.

Im Dentalbereich ist die Verwendung von Füllstoffen bekannt, deren Oberflächen mit polymerisationsfähigen Gruppen modifiziert sind, so dass diese bei der Aushärtung des Materials durch Copolymerisation kovalent mit der Polymermatrix (beispielsweise einer Methacrylatmatrix) verbunden werden. Silikatische Füllstoffe können zu diesem Zweck beispielsweise mit vorhydrolysierten (Meth)acryloxyalkyltrialkoxysilanen silanisiert werden (vgl. z.B. für Füllungs- und Befestigungsmaterialien DE 40 29 230, oder für Beschichtungen US 2002/0065337). Nichtsilikatische Füllstoffe wie z.B. Zirkonoxid können beispielsweise durch methacrylatmodifizierte Polyethercarbonsäuren (US 6,387,981) oder (Meth)acryloyloxyalkyldihydrogenphosphate (US 6,417,244) oberflächenmodifiziert werden.

Durch die Verwendung solcher Füllstoffe können die mechanischen Eigenschaften von Dentalmaterialien verbessert werden. Es hat sich jedoch gezeigt, dass bekannte Dentalmaterialien eine in vielen Fällen nicht optimale Haftung auf dem Zahnmaterial aufweisen.

Mit Aldehyd- oder Säuregruppen oberflächenfunktionalisierte Silikatmaterialien finden in der Molekularbiologie oder der Affinitätschromatographie beispielsweise zur Immobilisierung von Proteinen und Polypeptiden Verwendung. Dabei erfolgt z.B. die Herstellung von mit CHO-Gruppen funktionalisierten SiO₂-Partikeln oder SiO₂-Nanoröhren über eine Umsetzung mit aldehydgruppenhaltigen Silanen, wie z.B. Trimethoxysilylbutyraldehyd oder Trimethoxysilylpropionaldehyd (vgl. M.T. Dulay et al., Analyt. Chem. 77 (2005) 4604-4610; G. MacBeath, S. L. Schreiber, Science 289 (2000) 1760-1763; W. Clarke et al., J. Chromatography A 2000 (888) 13-22; S. B. Lee et al., Science 296 (2002) 2198-2200).

WO 2004/069400 beschreibt ein Verfahren zur Herstellung von Funktionskolloiden, bei dem Partikel in einem Dispergiermittel in Anwesenheit eines Modifizierungsmittels mechanisch reaktivzerkleinert werden, so dass das Modifizierungsmittel zumindest teilweise an die zerkleinerten Kolloidpartikel chemisch gebunden wird. Die Funktionskolloide können zur Herstellung von keramischen Formkörpern, Folien, Membranen und Beschichtungen verwendet werden.

WO 84/00375 beschreibt polymerisierbare Harze, die mineralische Füllstoffe mit darauf aufgepfropften Substituenten mit copolymerisierbaren funktionellen Gruppen enthalten und die in Schichten ausgehärtet werden, um quervernetzte Harzbeschichtungen zu erhalten.

Der Erfindung liegt die Aufgabe zugrunde, Füllstoffe bereitzustellen, die sich in verschiedenste Harz- bzw. Polymermatrixsysteme einfach einarbeiten lassen und zur Herstellung von dentalen Adhäsiven, Zementen, Kompositen oder Beschichtungen geeignet sind, gute mechanische Eigenschaften haben und eine verbesserte Haftung auf der Zahnhartsubstanz zeigen.

Die Aufgabe wird erfindungsgemäß durch die polymerisierbare Zusammensetzung gemäß den Ansprüchen 1 bis 16 gelöst. Die Erfindung betrifft auch das Verfahren zur Herstellung einer solchen Zusammensetzung gemäß den Ansprüchen 17 bis 20 sowie die Verwendung einer polymerisierbaren Zusammensetzung bzw. eines oberflächenmodifizierten Füllstoffs gemäß den Ansprüchen 21 bis 24 und 25 bis 27.

Die erfindungsgemäße polymerisierbare Zusammensetzung enthält mindestens einen mit Gruppen der Formel (I) oberflächenfunktionalisierten Füllstoff, bei dem Gruppen der Formel (I)

(A)a-Z-Y-R²-SiR¹₃₋ₘ-(O---)ₘ (I),

in der
- R¹: für C₁-C₁₅-Alkyl, C₂-C₅-Alkenyl oder Phenyl steht,
- R²: entfällt oder für einen linearen oder verzweigten C₁-C₆- Alkylenrest steht,
- Y: entfällt oder für eine Ether-, Thioether-, Amid-, Ester- oder Urethangruppe steht, Z entfällt oder für einen mindestens zweiwertigen linearen oder verzweigten aliphatischen Rest mit 2 bis 20 Kohlen- stoffatomen, der durch eine oder mehrere Ether-, Thio- ether-, Amid- oder Estergruppen unterbrochen sein kann und eine oder mehrere cycloaliphatische Gruppen mit mindestens 3 Kohlenstoffatomen und/oder eine oder mehrere aromatische Gruppen mit mindestens 6 Kohlenstoffatomen enthalten kann, einen mindestens zweiwertigen cycloaliphatischen Rest mit mindestens 3 Kohlenstoffatomen oder einen mindestens zwei- wertigen aromatischen Rest mit mindestens 6 Kohlenstoff- atomen steht,
- A: jeweils unabhängig für -COOH, -P(O)(OH)₂, -O-P(O)(OH)₂, -SO₂OH, -C(O)-O-C(O)-, -CHO, -NH-C(O)-CHO, -C(O)-CHO, -C(O)-CH₂-C(O)-CH₃, -N=C=O oder -O-C(O)-CH₂-C(O)-CH₃ steht,
- a: 1 bis 6 ist und
- m: 1 bis 3 ist,
wobei R² und Z nicht beide entfallen können,
wobei R² und Z jeweils nur entfallen können, wenn zugleich auch Y entfällt, und
wobei a 1 ist, wenn Z entfällt,
über mindestens ein an das Siliciumatom der Gruppe der Formel (I) gebundenes Sauerstoffatom mit dem Füllstoff verknüpft sind.

Der Begriff "verknüpft" bezieht sich hier auf eine chemische Bindung, vorzugsweise eine kovalente chemische Bindung.

Der Hinweis, dass ein Rest durch eine Gruppe wie beispielsweise eine Ethergruppe unterbrochen sein kann, ist so zu verstehen, dass die Gruppe in die Kohlenstoffkette des Restes eingeschoben wird, d.h. beidseitig von Kohlenstoffatomen begrenzt wird. Die Anzahl dieser Gruppen ist daher um mindestens 1 kleiner als die Zahl der Kohlenstoffatome, und die Gruppen können nicht endständig sein. Erfindungsgemäß sind Reste, die nicht durch die genannten Gruppen unterbrochen sind, bevorzugt.

Wenn A eine zweiwertige Gruppe, insbesondere -C(O)-O-C(O)-ist, sind die beiden endständigen Kohlenstoffatome dieser Gruppe jeweils an unterschiedliche Kohlenstoffatome der Gruppe Z gebunden. Wenn die Gruppe der Formel (I) mehr als eine Gruppe A enthält, können die mehreren Gruppen A jeweils an dasselbe und/oder vorzugsweise an unterschiedliche Kohlenstoffatome der Gruppe Z gebunden sein.

Erfindungsgemäß werden nur solche Verbindungen in Erwägung gezogen, die in mit der chemischen Valenzlehre vereinbar sind.

Es wurde überraschend gefunden, dass die erfindungsgemäßen Zusammensetzungen, die mit Gruppen der Formel (I) oberflächenfunktionalisierten Füllstoff enthalten, insbesondere als Dentalmaterialien geeignet sind, die sich durch eine verbesserte Haftung auf dem Zahnhartmaterial, insbesondere eine verbesserte Scherhaftfestigkeit auf Dentin und Zahnschmelz auszeichnen. Ohne Beschränkung auf eine bestimmte Theorie wird davon ausgegangen, dass mit Gruppen der Formel (I) oberflächenfunktionalisierte Füllstoffe über die funktionellen Gruppen A mit Hydroxylapatit und/oder Kollagen der Zahnhartsubstanz kovalente Bindungen eingehen können. Insbesondere können Säuregruppen mit Hydroxylapatit und Carbonsäureanhydride bzw. Aldehyde mit Kollagen reagieren.

Bevorzugt steht A jeweils unabhängig für -COOH, -P(O)(OH)₂, -O-P(O)(OH)₂, -SO₂OH, -CHO, -NH-C(O)-CHO, -C(O)-CHO, -C(O)-CH₂-C(O)-CH₃ oder -O-C(O)-CH₂-C(O)-CH₃.

Eine bevorzugte Ausführungsform der polymerisierbaren Zusammensetzung ist dadurch gekennzeichnet, dass
- R¹: für C₁-C₆-Alkyl oder Phenyl steht,
- R²: für lineares oder verzweigtes C₁-C₃-Alkylen steht,
- Y: entfällt oder für eine Ether-, Thioether-, Ester- oder Ur- ethangruppe steht,
- Z: entfällt oder für einen mindestens zweiwertigen linearen oder verzweigten aliphatischen Rest mit 2 bis 20 Kohlen- stoffatomen, der durch eine oder mehrere Ether-, Thio- ether-, Amid- oder Estergruppen unterbrochen sein kann und eine oder mehrere cycloaliphatische Gruppen mit mindestens 3 Kohlenstoffatomen und/oder eine oder mehrere aromatische Gruppen mit mindestens 6 Kohlenstoffatomen enthalten kann, einen mindestens zweiwertigen cycloaliphatischen Rest mit mindestens 3 Kohlenstoffatomen oder einen mindestens zwei- wertigen aromatischen Rest mit mindestens 6 Kohlenstoff- atomen steht,
- A: jeweils unabhängig für -COOH, -P(O)(OH)₂, -O-P(O)(OH)₂, -SO₂OH, -CHO, -NH-C(O)-CHO oder -O-C(O)-CH₂-C(O)-CH₃ steht,
- a: 1 bis 3 ist und
- m: 1 bis 3 ist.

Besonders bevorzugt steht R¹ für C₁-C₃-Alkyl, am meisten bevorzugt für Methyl.

Besonders bevorzugt steht R² für C₁-C₃-Alkylen.

Besonders bevorzugt steht Y für eine Ether- oder Thioethergruppe.

Besonders bevorzugt steht Z für einen mindestens zweiwertigen linearen oder verzweigten aliphatischen Rest mit 2 bis 10 Kohlenstoffatomen, der durch eine oder mehrere Ether- oder Estergruppen unterbrochen sein kann und eine oder mehrere cycloaliphatische Gruppen mit mindestens 3 Kohlenstoffatomen und/oder eine oder mehrere aromatische Gruppen mit mindestens 6 Kohlenstoffatomen enthalten kann, einen mindestens zweiwertigen cycloaliphatischen Rest mit mindestens 3 Kohlenstoffatomen oder einen mindestens zweiwertigen aromatischen Rest mit mindestens 6 Kohlenstoffatomen.

Besonders bevorzugt steht A jeweils unabhängig für -P(O)(OH)₂, -O-P(O)(OH)₂, -CHO oder -NH-C(0)-CHO.

Besonders bevorzugt ist a 1 oder 2.

Bei den genannten Alkyl- und Alkylenresten handelt es sich vorzugsweise um lineare Gruppen.

Die erfindungsgemäßen Zusammensetzungen enthalten mindestens einen Füllstoff, dessen Oberfläche funktionalisiert ist. Als Füllstoff eignen sich insbesondere anorganische Partikel und Fasern. Bevorzugt werden als Füllstoff partikuläre Materialien mit einer mittleren Partikelgröße von 1 nm bis 10 µm, vorzugsweise von 5 nm bis 5 µm verwendet. Der Begriff mittlere Partikelgröße bezieht sich hier auf das Volumenmittel.

Bevorzugte Füllstoffe sind anorganische, vorzugsweise amorphe Materialien. Besonders bevorzugt sind monodisperse, nanopartikuläre Füllstoffe, vorzugsweise auf Basis von SiO₂, wie pyrogene Kieselsäure oder Fällungskieselsäure, Oxiden der Elemente Zr, Ti, A1, Y, La, Ce und/oder Yb und deren Mischoxiden mit SiO₂. Es ist bevorzugt, dass der Füllstoff eine mittlere Partikelgröße von 5 bis 200 nm, besonders bevorzugt 10 bis 100 nm, ganz besonders bevorzugt 10 bis 50 nm aufweist.

Die Gruppen der Formel (I) können allgemein abgeleitet werden von Silanen der Formel (II)

(A)ₐ-Z-Y-R²-SiXₙR¹₃₋ₙ (II),

in der
- X: für Halogen, Hydroxy, C₁-C₅-Alkoxy oder C₁-C₃-Acyloxy steht,
- A: jeweils unabhängig für -COOH, -P(O) (OR³)₂, -O-P(O)(OR³)₂, -SO₂OH, -C(O)-O-C(O)-, -CHO, -NH-C(O)-CHO, -C(O)-CHO, -C(O)-CH₂-C(O)-CH₃, -N=C=O oder -O-C(O)-CH₂-C(O)-CH₃ steht,
- R³: jeweils unabhängig für H oder C₁-C₅-Alkoxy steht,
- n: 1 bis 3 ist
und die übrigen Bedeutungen wie oben für Formel (I) definiert sind.

Bevorzugt steht A jeweils unabhängig für -COOH, -P(O)(OR³)₂, -O-P(O) (OR³)₂, -SO₂OH, -CHO, -NH-C(O)-CHO oder -O-C(O)-CH₂-C(O)-CH₃.

Besonders bevorzugt steht X für Halogen oder C₁-C₃-Alkoxy, insbesondere Cl, Methoxy, Ethoxy oder n-Propoxy, am meisten bevorzugt Methoxy.

Besonders bevorzugt steht A jeweils unabhängig für -P(O)(OR³)₂, -O-P(O)(OR³)₂, -CHO oder -NH-C(O)-CHO.

Besonders bevorzugt steht R³ für H oder C₁-C₃-Alkoxy, am meisten bevorzugt H, Methoxy oder Ethoxy.

Beispiele für Silane gemäß Formel (II) sind u.a.:

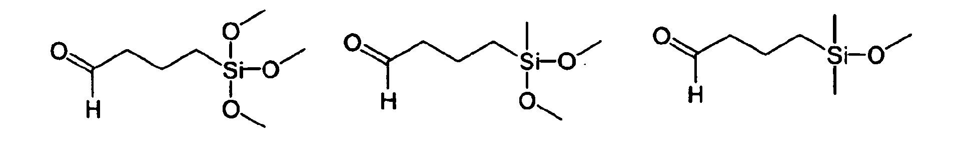

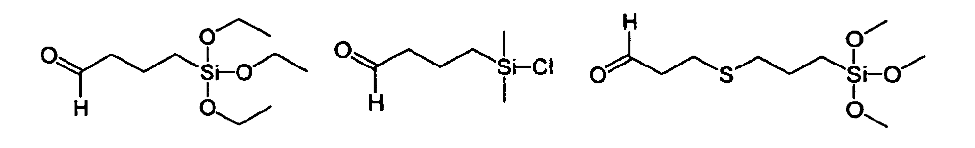

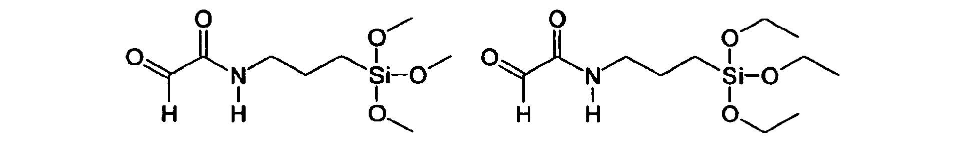

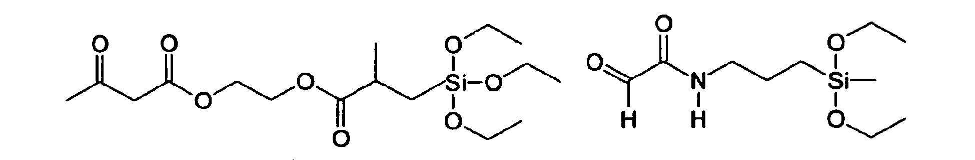

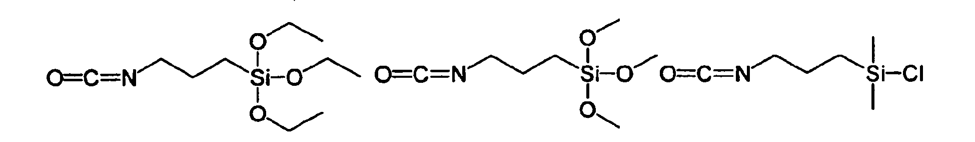

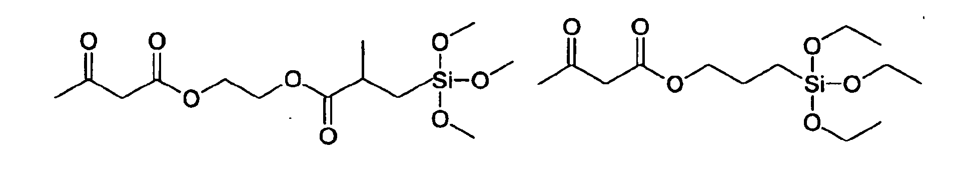

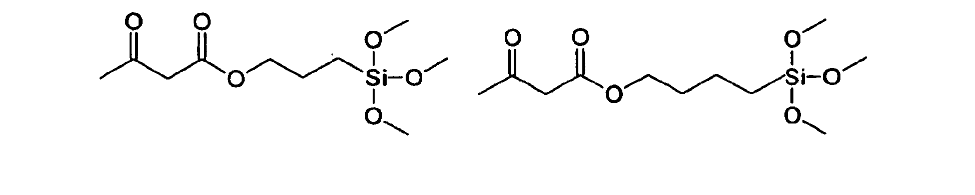

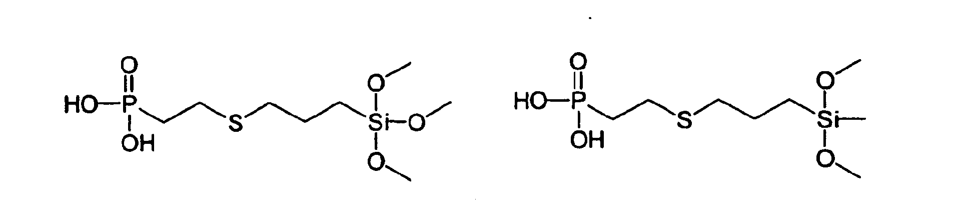

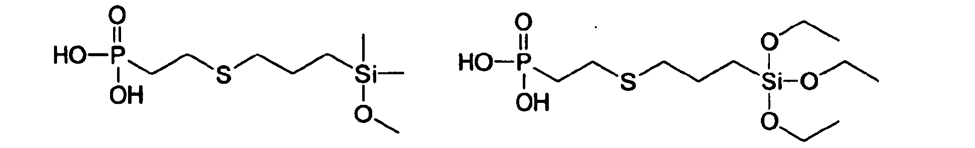

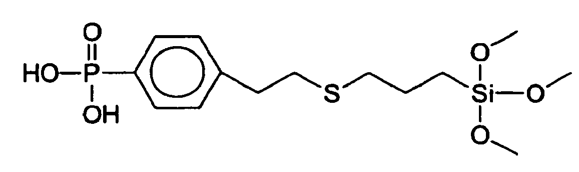

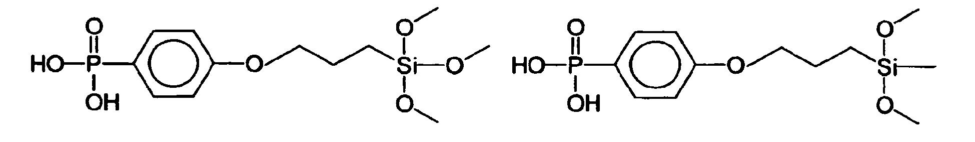

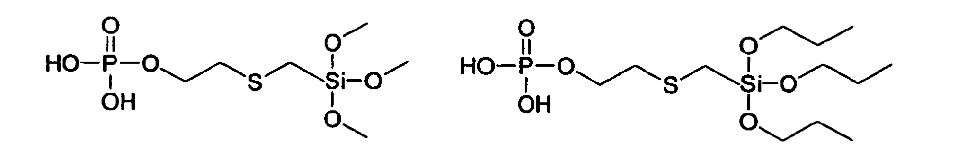

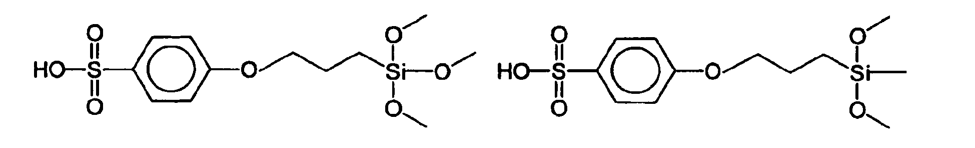

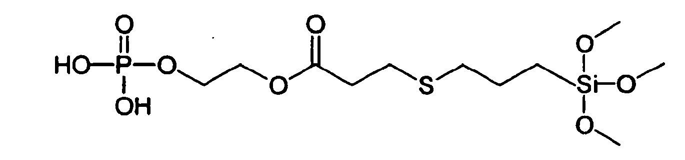

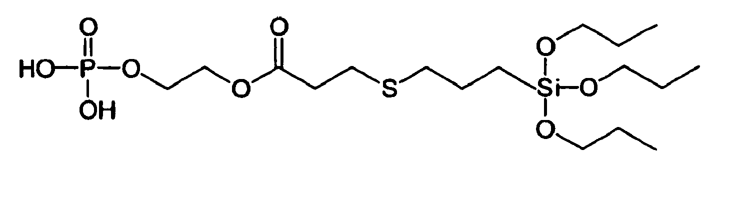

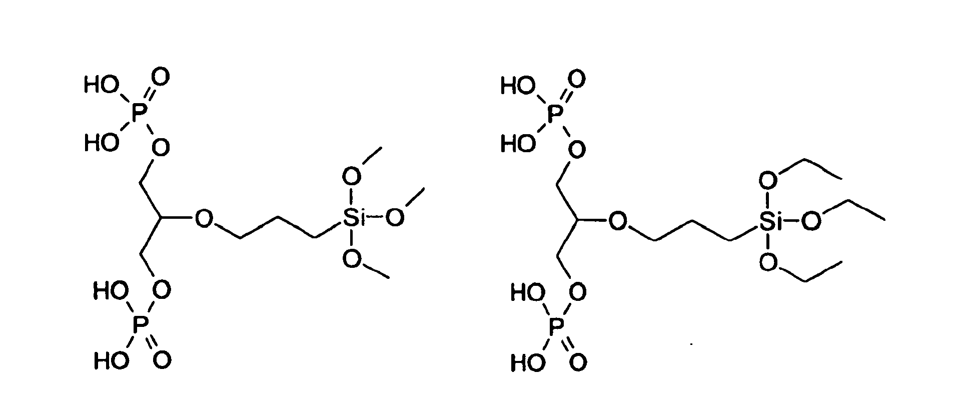

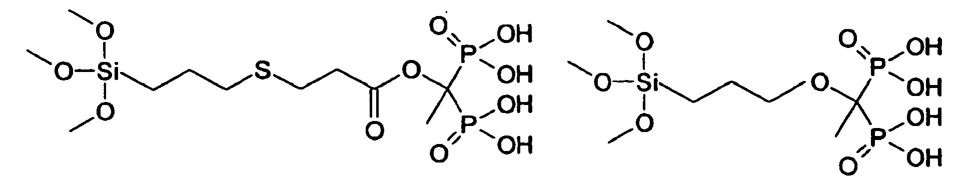

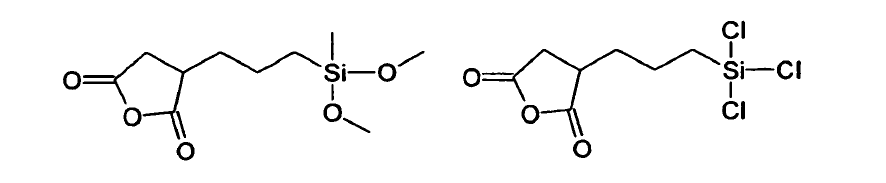

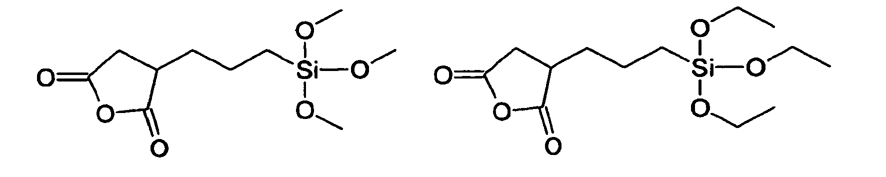

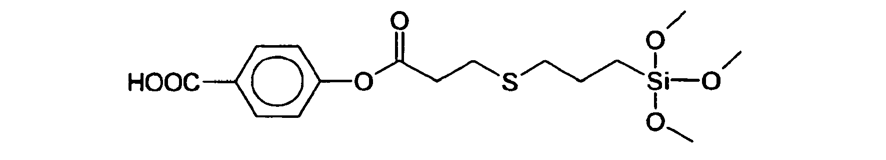

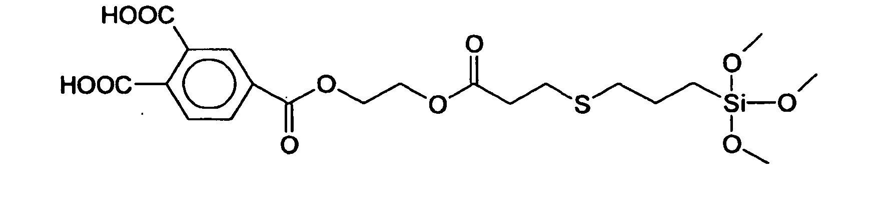

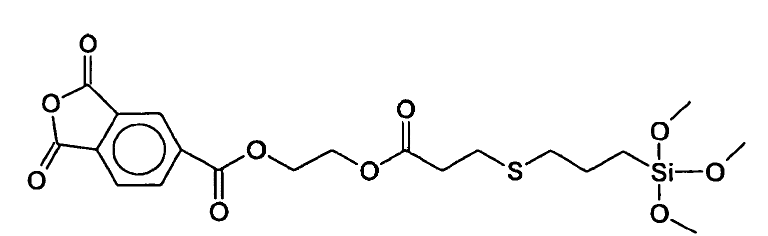

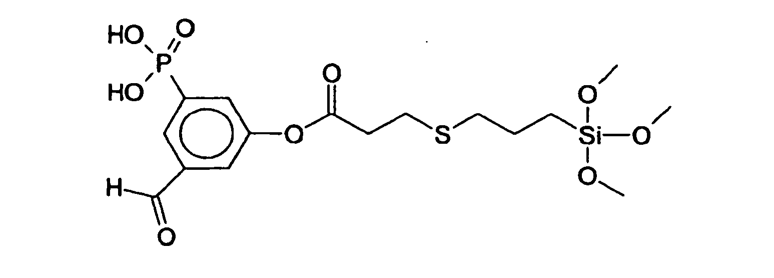

Zusammensetzungen, die mindestens einen mit Gruppen der Formel (I) oberflächenfunktionalisierten Füllstoff aufweisen, bei dem sich die Gruppen der Formel (I) von einem der oben genannten Silane der Formel (II) ableiten, sind erfindungsgemäß besonders bevorzugt.

Funktionalisierte Silane gemäß Formel (II) sind teilweise bekannt oder kommerziell erhältlich. Beispielsweise sind folgende Silane kommerziell erhältlich:

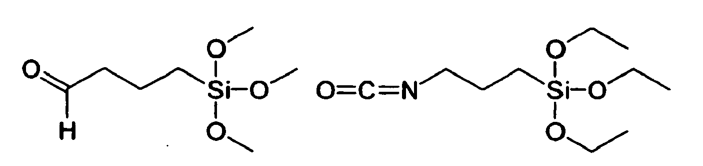

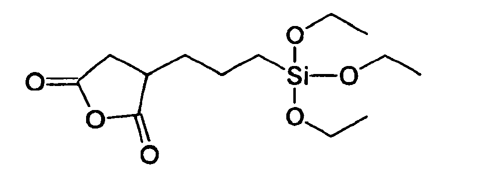

Silane gemäß Formel (II) lassen sich grundsätzlich analog zu aus der Siliciumchemie (beispielweise M. A. Brook, Silicon in Organic, Organometallic, and Polymer Chemistry, John Wiley & Sons Inc., New York etc. 1999) und organischen Chemie (beispielsweise W. Walter, W. Franke, Bayer-Walter Lehrbuch der organischen Chemie, 24. Aufl., S. Hirzel Verlag, Stuttgart und Leipzig 2004; Autorenkollektiv, Organikum, 21. Aufl., Wiley-VCH, Weinheim etc. 2001) bekannten Methoden herstellen.

Eine Synthesemethode ist beispielsweise die Verknüpfung von Si-H- und vinylgruppenhaltigen Verbindungen durch Hydrosilylierung:

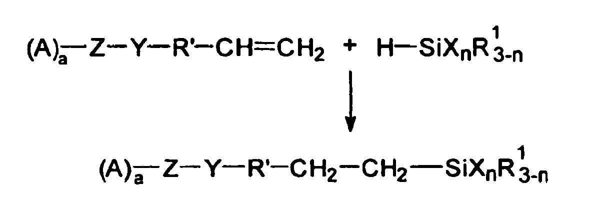

Konkretes Beispiel zur Herstellung eines aldehydgruppenhaltigen Silans: Hydrosilylierung von Allylalkohol mit Trimethoxysilan und nachfolgende Oxidation der primären OH-Gruppe zur Aldehydgruppe:

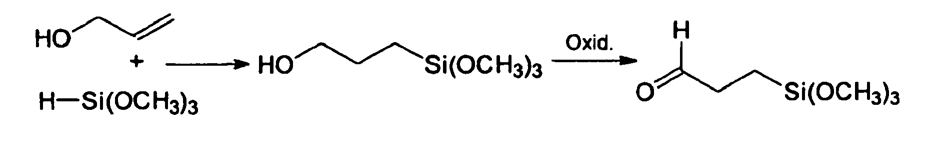

Eine andere Synthesemethode stellt die Thiol-En-Addition dar:

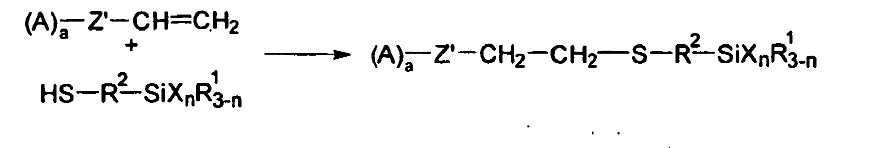

Konkretes Beispiel zur Herstellung eines aldehydgruppenhaltigen Silans: Thiol-En-Addition von Acrolein mit 3-Mercaptopropyltrimethoxysilan:

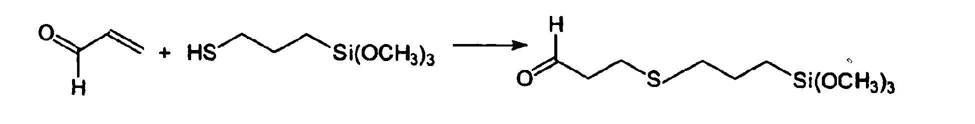

Eine weitere Synthesemethode ist die Addition an Isocyanatgruppen:

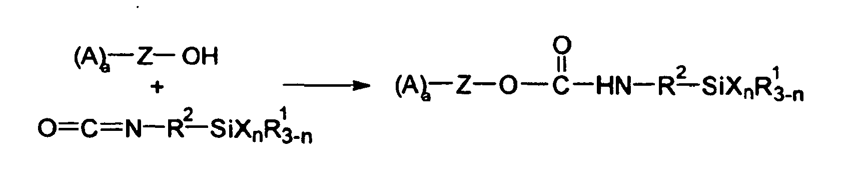

Konkretes Beispiel zur Herstellung eines aldehydgruppenhaltigen Silans: Umsetzung von 4-Hydroxymethylbenzaldehyd mit 3-Isocyanatopropyltriethoxysilan:

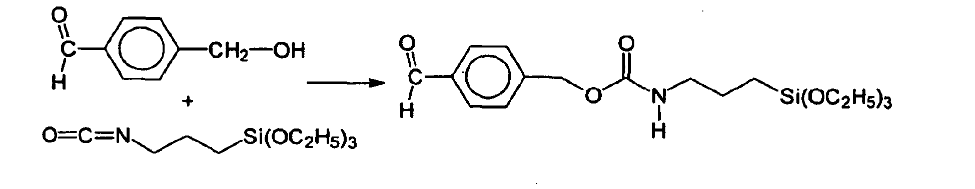

Neben den oben beispielhaft erwähnten Synthesemethoden zur Herstellung der Silane gemäß Formel (II) sind dem Fachmann weitere Methoden allgemein bekannt.

Mit Gruppen der Formel (I) oberflächenmodifizierte Füllstoffe sind insbesondere durch Umsetzung des Füllstoffs mit einem Silan erhältlich. Im Falle von silikatischen Füllstoffen kommt es dabei zur Ausbildung von stabilen Siloxanbindungen zwischen Silanol-Gruppen auf der Oberfläche des Füllstoffs und Siliciumatomen des Silans.

In einer Ausführungsform wird ein mit Gruppen der Formel (I) oberflächenmodifizierter Füllstoff dadurch erhalten, dass der Füllstoff mit mindestens einem Silan der Formel (II) umgesetzt wird.

In einer anderen Ausführungsform wird in einem ersten Schritt der Füllstoff mit einem Silan umgesetzt, das eine Vorstufe eines Silans der Formel (II) darstellt, und das erhaltene Produkt nachfolgend in einem oder mehreren Schritten zu einem mit Gruppen der Formel (I) oberflächenfunktionalisierten Füllstoff umgesetzt. Dabei sind Silane, die eine Vorstufe eines Silans der Formel (II) gemäß einem der oben diskutierten Verfahren darstellen, besonders bevorzugt.

Beispielsweise kann in einem ersten Schritt eine Silylierung des Füllstoffs mit einem Hydrosilan der Formel H-SiXₙR¹₃₋ₙ erfolgen und das so erhaltene Produkt anschließend in einer Hydrosilylierung mit einer vinylgruppenhaltigen Verbindung der Formel (A)ₐ-Z-Y-R'-CH=CH₂ umgesetzt werden. Konkretes Beispiel zur Herstellung eines aldehydgruppenhaltigen Füllstoffs: Silylierung des Füllstoffs mit Trimethoxysilan, nachfolgende Umsetzung des Produkts mit Allylalkohol und abschließende Oxidation der primären OH-Gruppe zur Aldehydgruppe.

Gemäß einer anderen Synthesemethode wird der Füllstoff in einem ersten Schritt mit einem Mercaptoalkylsilan der Formel HS-R²-SiXₙR¹₃₋ₙ silanisiert und das so erhaltene Produkt in einer Thiol-En-Addition mit einer vinylgruppenhaltigen Verbindung der Formel (A)ₐ-Z'-CH=CH₂ umgesetzt. Konkretes Beispiel zur Herstellung eines aldehydgruppenhaltigen Füllstoffs: Silylierung des Füllstoffs mit 3-Mercaptopropyltrimethoxysilan und nachfolgende Umsetzung des Produkts mit Acrolein.

Gemäß einer weiteren Synthesemethode wird in einem ersten Schritt der Füllstoff mit einem Isocyanatoalkylsilan der Formel O=C=N-R²-SiXₙR¹₃₋ₙ silanisiert und das so erhaltene Produkt mit einem Alkohol der Formel (A)ₐ-Z-OH umgesetzt. Konkretes Beispiel zur Herstellung eines aldehydgruppenhaltigen Füllstoffs: Silanisierung des Füllstoffs mit 3-Isocyanatopropyltriethoxysilan und nachfolgende Umsetzung des Produkts mit 4-Hydroxymethylbenzaldehyd.

Im Falle der Funktionalisierung mit Phosphonsäuregruppen -P(O)(OH)₂ oder Dihydrogenphosphatgruppen -O-P(O)(OH)₂ kann auch so vorgegangen werden, dass zunächst eine Oberflächenfunktionalisierung des Füllstoffs mit einem Silan erfolgt, das mindestens eine Phosphonsäureestergruppe -P(O)(OR³)₂ oder Phosphorsäureestergruppe -O-P(O)(OR³)₂ enthält. Anschließend wird eine Freisetzung der entsprechenden Säuregruppe(n) durch Hydrolyse oder Alkoholyse durchgeführt.

Beispielsweise kann ein Füllstoff zur Funktionalisierung mit Phosphonsäuregruppen in einem ersten Syntheseschritt mit kommerziell erhältlichem Diethoxyphosphorylethyltriethoxysilan (R³ = Et) umgesetzt werden, wobei eine Verknüpfung des Silans mit dem Füllstoff erfolgt. Im zweiten Schritt wird dann die Phosphonsäuregruppe durch Hydrolyse der Phosphonsäureestergruppe freigesetzt. Dies ist beispielsweise für den Fall eines silikatischen Füllstoffs im folgenden Schema dargestellt:

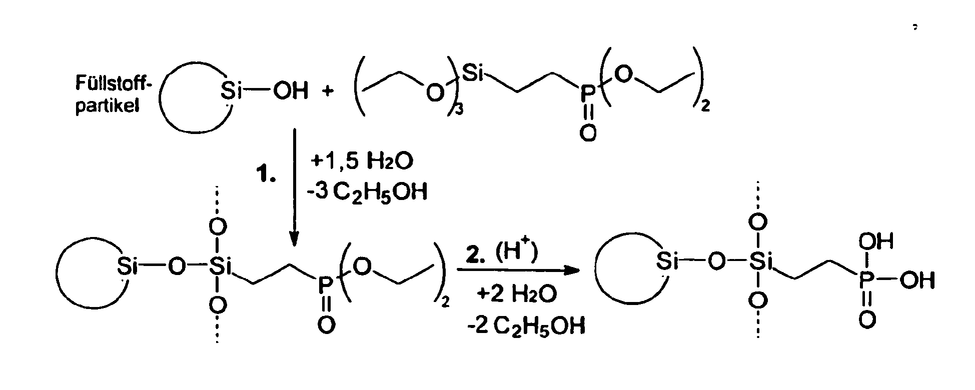

Die Herstellung eines mit Gruppen der Formel (I) oberflächenfunktionalisierten Füllstoffs durch Umsetzung eines Füllstoffs mit einem Silan kann auf unterschiedlichen Wegen erfolgen. Beispielsweise kann ein flüssiges Silan direkt mit Füllstoff vermischt und anschließend zur Abtrennung von Kondensationsprodukten getrocknet werden.

In einer anderen Ausführungsform wird ein Füllstoff in einer Lösung des Silans in einem geeigneten Lösungsmittel dispergiert. Dabei lässt sich die Wechselwirkung des Silans mit der Füllstoffoberfläche durch die Polarität des Lösungsmittels beeinflussen. Es hat sich gezeigt, dass ein solches Verfahren zu einer besseren Benetzung der Füllstoffoberfläche führt und insbesondere bei sehr feinteiligen Füllstoffen mit einer spezifischen Oberfläche größer 30 m²/g, insbesondere größer 40 m²/g vorteilhaft ist. Beispiele für geeignete Lösungsmittel sind C₁-C₆-Alkanole, wie z.B. Ethanol oder Isopropanol, cyclische Ether, wie z.B. Tetrahydrofuran oder Dioxan, aliphatische Ester, wie z.B. Ethylacetat oder Butylacetat, aliphatische Kohlenwasserstoffe, wie z.B. Hexan, und cycloaliphatische Kohlenwasserstoffe, wie z.B. Cyclohexan.

Nach Beendigung der Reaktion wird der Füllstoff abgetrennt, gegebenenfalls einmal oder mehrmals mit demselben und/oder einem anderen Lösungsmittel gewaschen, gegebenenfalls einer Wärmebehandlung unterzogen, gegebenenfalls nochmals gewaschen und dann getrocknet. Nach der Oberflächenfunktionalisierung wird der Füllstoff gegebenenfalls gemahlen. Dies kann insbesondere bei Füllstoffen vorteilhaft sein, die zur Agglomeratbildung neigen.

Insbesondere bei der Verwendung von Nanopartikeln als Füllstoff kann es vorteilhaft sein, den Füllstoff in Form eines Organosols einzusetzen. Der Begriff "Organosol" bezeichnet hier insbesondere kolloidale Suspensionen, in denen die kontinuierliche Phase eine organische Verbindung, insbesondere ein organisches Lösungsmittel oder ein bei Raumtemperatur flüssiges polymerisierbares Monomer ist. Beispiele für geeignete polymerisierbare Monomere sind wie unten beschrieben.

Bei der Umsetzung eines Füllstoffs mit einem Silan hängt der Grad der Oberflächenfunktionalisierung unter anderem ab von der Füllstoffmenge bzw. der spezifischen Oberfläche des Füllstoffs, von der Menge und Struktur des Silans, der Reaktionszeit, der Temperatur, der Art des verwendeten Katalysators und von der Füllstoffvorbehandlung, wie z.B. einer Vortrocknung. Die verschiedenen Einflussfaktoren sind allgemein vor allem bei der Silanisierung von SiO₂ sehr gut untersucht (vgl. E. P. Plueddemann, "Silane Coupling Agents", Plenum Press, 2nd Ed., New York und London, 1991; A. Guillet, Macromol. Symp. 194 (2003) 63).

Erfindungsgemäß sind mit Gruppen der Formel (I) oberflächenfunktionalisierte Füllstoffe bevorzugt, die dadurch erhalten werden können, dass ein Füllstoff mit mindestens 0,01 mmol, vorzugsweise 0,1 - 5 mmol, besonders bevorzugt 0,5 bis 2 mmol eines geeigneten Silans pro Gramm des Füllstoffs umgesetzt wird. Dabei sind Silane der Formel (II) und Silane, die eine Vorstufe eines Silans der Formel (II) darstellen, wie oben beschrieben, besonders bevorzugt.

Der Grad der Oberflächenfunktionalisierung des mit Gruppen der Formel (I) oberflächenfunktionalisierten Füllstoffs lässt sich beispielsweise mittels Elementaranalyse bestimmen. Bei Gruppen der Formel (I), die Phosphor und/oder Schwefel enthalten, kann insbesondere der Gehalt des oberflächenfunktionalisierten Füllstoffs an einem dieser Elemente zur Bestimmung des Funktionalisierungsgrads verwendet werden.

Es ist bevorzugt, dass der mit Gruppen der Formel (I) oberflächenfunktionalisierte Füllstoff mindestens 0,01 mmol, vorzugsweise 0,05 - 2 mmol, besonders bevorzugt 0,1 bis 1 mmol Gruppen der Formel (I) pro Gramm des Füllstoffs enthält. Im Falle von Füllstoffen auf Basis von SiO₂ ist es insbesondere bevorzugt, dass der mit Gruppen der Formel (I) oberflächenfunktionalisierte Füllstoff mindestens 0,01 mmol, vorzugsweise 0,05 - 2 mmol, besonders bevorzugt 0,1 bis 1 mmol Gruppen der Formel (I) pro Gramm SiO₂ enthält.

In den erfindungsgemäßen Zusammensetzungen kann der mit Gruppen der Formel (I) oberflächenfunktionalisierte Füllstoff zusätzlich mit weiteren Gruppen modifiziert sein. Der Begriff "weitere Gruppe" bezeichnet hier eine Gruppe, die nicht die Formel (I) aufweist. Beispielweise kann der mit Gruppen der Formel (I) oberflächenfunktionalisierte Füllstoff zusätzlich mit polymerisationsfähigen und/oder nichtfunktionalisierten Gruppen modifiziert sein. Bevorzugte polymerisationsfähige Gruppen sind Gruppen, die mindestens eine (Meth)acrylesterund/oder (Meth)acrylamidfunktionalität aufweisen, insbesondere (Meth)acryloyloxyalkylsilylgruppen bzw. (Meth)acrylamidoalkylsilylgruppen. Unter Alkyl werden bevorzugt Reste mit 1 bis 6, insbesondere 1 bis 3 Kohlenstoffatomen verstanden. Unter nichtfunktionalisierten Gruppen werden solche Gruppen verstanden, die nicht die Formel (I) aufweisen und nicht polymerisationsfähig sind. Eine zusätzliche Oberflächenmodifizierung des Füllstoffs, beispielsweise mit polymerisationsfähigen Gruppen, kann insbesondere die Einarbeitbarkeit des Füllstoffs in die erfindungsgemäßen Zusammensetzungen und die mechanischen Eigenschaften von daraus hergestellten Dentalmaterialien verbessern.

Mit Gruppen der Formel (I) oberflächenfunktionalisierte Füllstoffe, die zusätzlich mit weiteren Gruppen modifiziert sind, sind insbesondere dadurch zugänglich, dass der Füllstoff vor, nach oder zusammen mit der Oberflächenfunktionalisierung mit mindestens einer Gruppe der Formel (I) mit mindestens einem weiteren Silan umgesetzt wird. Vorzugsweise lässt sich bei der Oberflächenfunktionalisierung des Füllstoffs eine Mischung von mindestens einem Silan der Formel (II) mit mindestens einem weiteren Silan einsetzen. Eine andere Variante besteht darin, vor oder nach der Oberflächenmodifizierung eines Füllstoffs mit mindestens einem Silan der Formel (II) eine Silanisierung des Füllstoffs mit einem oder mehreren polymerisationsfähigen und/oder nichtfunktionalisierten weiteren Silanen durchzuführen.

Beispiele für geeignete polymerisationsfähige Silane sind (Meth)acryloyloxyalkyltrialkoxysilane, wie z.B. 3-Methacryloyloxypropyltrimethoxysilan, 3-Acryloyloxypropyltrimethoxysilan, 3-Methacryloyloxypropylmethyldimethoxysilan, 3-Methacryloyloxypropyldimethylmethoxysilan, 3-Acryloyloxypropylmethyldimethoxysilan oder 3-Acryloyloxypropyldimethylmethoxysilan. Polymerisationsfähige Silane, die zwei Methacrylatreste tragen, lassen sich z.B. durch Umsetzung von Glycerindimethacrylat mit 3-Isocyanatopropyltriethoxysilan oder 3-(Methyldiethoxysilyl)-propylbernsteinsäureanhydrid bzw. mit Glutarsäureanhydrid und anschließend mit 3-Aminopropyltriethoxysilan einfach herstellen. Geeignet sind auch polymerisationsfähige (Meth)acrylamidoalkyltrialkoxysilane, wie z.B. 3-(N-Methacryloylamino)-propyltrimethoxysilan, 3-(N-Acryloylamino)-propyltrimethoxysilan, 3-(N-Methacryloylamino)-propyltriethoxysilan, 3-(N-Methacryloyl-N-ethyl-amino)-propyltrimethoxysilan, 3-(N-Methacryloyl-N-ethyl-amino)-propyltrimethoxysilan, 3-(N-Acryloyl-N-ethylamino)-propyltrimethoxysilan, 3-(N-Methacryloyl-N-methyl-amino)-propyltrimethoxysilan oder 3-(N-Acryloyl-N-methyl-amino)-propyltrimethoxysilan, deren polymerisationsfähige (Meth)acrylamidgruppen besonders gute Hydrolysestabilität aufweisen.

Neben mit Gruppen der Formel (I) oberflächenfunktionalisiertem Füllstoff enthalten die erfindungsgemäßen Zusammensetzungen mindestens ein polymerisierbares Monomer. Als polymerisierbare Monomere eignen sich besonders radikalisch polymerisierbare Monomere.

Diese radikalisch polymerisierbaren Monomere können eine oder mehrere radikalisch polymerisierbare Gruppen aufweisen. Als radikalisch polymerisierbare Monomere sind bei Raumtemperatur flüssige Monomere bevorzugt, die sich als Verdünnermonomere eignen. Bevorzugt sind Monomere mit einer Viskosität von 0,01 bis 10 Pa·s bei Raumtemperatur, insbesondere mono- oder mehrfach funktionelle (Meth)acrylate. Besonders bevorzugt sind hydrolysestabile Verdünnermonomere, insbesondere Mono(meth)acrylate, wie z.B. Mesitylmethacrylat, 2-(Alkoxymethyl)acrylsäuren, wie z.B. 2-(Ethoxymethyl)acrylsäure und 2-(Hydroxymethyl)acrylsäure, N-mono-alkylsubstituierte Acrylamide, wie z.B. N-Ethylacrylamid oder N-(2-Hydroxyethyl)-acrylamid, N-mono-alkylsubstituierte Methacrylamide, wie z.B. N-Ethylmethacrylamid, N-(2-Hydroxyethyl)methacrylamid oder N-(5-Hydroxypentyl)methacrylamid, N,N-dialkylsubstituierte Acrylamide, wie z.B. N,N-Dimethylacrylamid oder N-Methyl-N-(2-hydroxyethyl)acrylamid, und N-Vinylpyrrolidon. Unter Alkyl werden bevorzugt Reste mit 1 bis 6, insbesondere 1 bis 3 Kohlenstoffatomen verstanden. Beispiele für weitere Verdünnermonomere sind Mono(meth)acrylate, wie z.B. Methyl-, Ethyl-, Butyl-, Benzyl-, Furfuryl- oder Phenyl(meth)acrylat.

Die erfindungsgemäßen Zusammensetzungen enthalten neben dem mit Gruppen der Formel (I) oberflächenfunktionalisierten Füllstoff vorzugsweise 0 bis 50 Gew.-%, vorzugsweise 5 bis 40 Gew.-% und ganz besonders bevorzugt 10 bis 30 Gew.-% Verdünnermonomer. Diese und, wenn nicht anders angegeben, alle anderen Prozentangaben beziehen sich auf die Gesamtmasse der Zusammensetzung.

Vorzugsweise enthalten die erfindungsgemäßen Zusammensetzungen mindestens ein Monomer mit 2 oder mehr, insbesondere 2 bis 5 radikalisch polymerisierbaren Gruppen. Monomere mit 2 oder mehr polymerisierbaren Gruppen wirken als Vernetzer und erhöhen so die mechanische Stabilität der gehärteten Zusammensetzungen.

Bevorzugte Vernetzermonomere sind hydrolysestabile Vernetzermonomere, insbesondere vernetzende Pyrrolidone, wie z.B. 1,6-Bis(3-vinyl-2-pyrrolidonyl)-hexan oder kommerziell zugängliche Bis(meth)acrylamide, wie z.B. Methylen- oder Ethylenbisacrylamid, N,N'-Diethyl-1,3-bis(acrylamido)-propan, 1,3-Bis(methacrylamido)-propan, 1,4-Bis(acrylamido)-butan, 1,4-Bis(acryloyl)-piperazin, 2,6-Dimethylen-4-oxa-heptan-1,7-dicarbonsäurebis-(propylamid), 1,6-Bis-(acrylamido)-2,2,4(2,4,4)-trimethylhexan und N,N'-Dimethyl-1,6-bis-(acrylamido)-hexan. Beispiele für weitere Vernetzer sind mehrfunktionelle (Meth)acrylate, wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethy-lendiisocyanat), Di-, Tri- oder Tetraethylenglycoldi(meth)acrylat, Trimethylolpropantri(meth)acrylat, Pentaerythrittetra(meth)acrylat, Butandioldi(meth)acrylat, 1,10-Decandioldi(meth)acrylat oder 1,12-Dodecandioldi(meth)acrylat.

Zusammensetzungen, die neben dem mit Gruppen der Formel (I) oberflächenfunktionalisierten Füllstoff 0 bis 45 Gew.-%, vorzugsweise 1 bis 30 Gew.-% und ganz besonders bevorzugt 5 bis 20 Gew.-% Vernetzermonomer enthalten, insbesondere Bis(meth)acrylamid, sind erfindungsgemäß besonders bevorzugt.

Gemäß einer weiteren bevorzugten Ausführungsform enthalten die Zusammensetzungen mindestens ein acides radikalisch polymerisierbares Monomer, d.h. ein Monomer mit einer oder mehreren aciden Gruppen, wie Carbonsäureanhydrid-, Carbonsäure-, Phosphorsäure-, Dihydrogenphosphat-, Phosphonsäure- und Sulfonsäuregruppen. Bevorzugte acide Gruppen sind Carbonsäure-, Phosphorsäure- und Phosphonsäuregruppen. Solche Monomere eignen sich als Adhäsivmonomere insbesondere für Schmelz/Dentin-Adhäsive oder selbsthaftende Komposite.

Besonders bevorzugte acide Monomere sind polymerisationsfähige Acrylatetherphosphonsäuren, wie z.B. 2-[4-(Dihydroxyphosphoryl)-2-oxa-butyl]-acrylsäureethylester, 2-[4-(Dihydroxyphosphoryl)-2-oxa-butyl]-acrylsäure oder 2-[4-(Dihydroxyphosphoryl)-2-oxa-butyl]-acrylsäure-2,4,6-trimethylphenylester, (Meth)acrylamidoalkylenphosphonsäuren oder (Meth)acrylamidoalkylenbisphosphonsäuren. Weiterhin sind als Adhäsivmonomere auch hydrolysestabile, polymerisationsfähige Dihydrogenphosphate wie (Meth)acrylamidoalkylenphosphate, (Meth)acrylamidocycloalkylenphosphate oder (Meth)acrylamidoarylendihydrogenphosphate, z.B. 2-(N-Acryloylamino)ethyldihydrogenphosphat, 2-(N-Methacryloylamino)ethyldihydrogenphosphat, 6-(N-Acryloylamino)hexyldihydrogenphosphat, 6-(N-Methacryloylamino)hexyldihydrogenphosphat, 4-(N-Acryloylamino)phenyldihydrogenphosphat, 4-(N-Methacryloylamino)phenyldihydrogenphosphat, 1,3-Bis-(N-acryloylamino)-propan-2-yl-dihydrogenphosphat, 1,3-Bis-(N-methacryloylamino)-propan-2-yl-dihydrogenphosphat, 1,3-Bis-(N-acryloyl-N-methyl-amino)-propan-2-yl-dihydrogenphosphat oder 1,3-Bis-(N-acryloyl-N-ethyl-amino)-propan-2-yl-dihydrogenphosphat besonders geeignet.

Zusammensetzungen, die neben dem mit Gruppen der Formel (I) oberflächenfunktionalisierten Füllstoff 1 bis 50 Gew.-%, vorzugsweise 5 bis 40 Gew.-% und ganz besonders bevorzugt 10 bis 30 Gew.-% acides Monomer enthalten, insbesondere acides Monomer mit Dihydrogenphosphat-, Phosphonsäure- und/oder Sulfonsäuregruppen, sind erfindungsgemäß besonders bevorzugt.

Neben dem mit Gruppen der Formel (I) oberflächenfunktionalisierten Füllstoff kann die erfindungsgemäße Zusammensetzung vorzugsweise mindestens einen weiteren Füllstoff enthalten, der nicht mit Gruppen der Formel (I) oberflächenmodifiziert ist. Beispiele für geeignete weitere Füllstoffe sind Füllstoffe, die nicht oberflächenmodifiziert sind, Füllstoffe, die mit polymerisierbaren Gruppen oberflächenmodifiziert sind, und Füllstoffe, die mit nichtfunktionalisierten Gruppen oberflächenmodifiziert sind. Bevorzugte polymerisationsfähige Gruppen sind Gruppen, die mindestens eine (Meth)acrylester- und/oder (Meth)acrylamidfunktionalität aufweisen, insbesondere (Meth)acryloyloxyalkylsilylgruppen bzw. (Meth)acrylamidoalkylsilylgruppen. Unter Alkyl werden bevorzugt Reste mit 1 bis 6, insbesondere 1 bis 3 Kohlenstoffatomen verstanden. Unter nichtfunktionalisierten Gruppen werden solche Gruppen verstanden, die nicht die Formel (I) aufweisen und nicht polymerisationsfähig sind. Solche oberflächenmodifizierten Füllstoffe sind insbesondere durch Silanisierung eines Füllstoffs mit geeigneten Silanen erhältlich. Beispielsweise können Füllstoffe, die mit polymerisierbaren Gruppen oberflächenmodifiziert sind, durch Silanisierung eines Füllstoffs mit mindestens einem der oben beschriebenen polymerisationsfähigen Silane erhalten werden.

Vorzugsweise enthält die erfindungsgemäße Zusammensetzung neben dem mit Gruppen der Formel (I) oberflächenfunktionalisierten Füllstoff 0 bis 40 Gew.-%, insbesondere 1 bis 30 Gew.-% weiteren Füllstoff, der nicht mit Gruppen der Formel (I) oberflächenmodifiziert ist.

Zur Initiierung der Polymerisation enthalten die erfindungsgemäßen Zusammensetzungen vorzugsweise einen Initiator für die radikalische Polymerisation, insbesondere für die photochemisch oder redoxinduzierte radikalische Polymerisation. Beispiele für geeignete Initiatoren für die Photopolymerisation sind Benzophenon, Benzoin sowie deren Derivate oder α-Diketone oder deren Derivate, wie 9,10-Phenanthrenchinon, 1-Phenylpropan-1,2-dion, Diacetyl oder 4,4'-Dichlorbenzil. Besonders bevorzugt werden Campherchinon und 2,2-Dimethoxy-2-phenylacetophenon und ganz besonders bevorzugt α-Diketone in Kombination mit Aminen als Reduktionsmitteln eingesetzt. Bevorzugte Amine sind 4-(N,N-Dimethylamino)-benzoesäureester, N,N-Dimethylaminoethylmethacrylat, N,N-Dimethyl-sym.-xylidin und Triethanolamin. Darüber hinaus sind auch Acylphosphine, wie z.B. 2,4,6-Trimethylbenzoyldiphenyl- oder Bis-(2,6-dichlorbenzoyl)-4-N-propylphenylphosphinoxid besonders geeignet.

Als Initiatoren für eine bei Raumtemperatur durchgeführte Polymerisation werden Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden und Reduktionsmitteln, wie z.B. Ascorbinsäure, Barbituraten oder Sulfinsäuren, besonders geeignet.

Zusammensetzungen, die neben dem oberflächenfunktionalisierten Füllstoff 0,1 bis 5,0 Gew.-%, vorzugsweise 0,2 bis 2,0 Gew.-% und ganz besonders bevorzugt 0,2 bis 1,0 Gew.-% Initiator für die radikalische Polymerisation enthalten, sind erfindungsgemäß besonders bevorzugt.

Die erfindungsgemäßen Zusammensetzungen können zudem Lösungsmittel, wie Wasser, Ethylacetat oder Ethanol, oder Lösungsmittelgemische enthalten. Dabei sind hydrolysestabile Lösungsmittel, wie Wasser oder Ethanol, oder Lösungsmittelgemische bevorzugt.

Darüber hinaus können die erfindungsgemäßen Zusammensetzungen weitere Additive enthalten, insbesondere Stabilisatoren, Aromastoffe, Farbmittel, mikrobiocide Wirkstoffe, fluoridionenabgebende Additive, optische Aufheller, Weichmacher und UV-Absorber.

Erfindungsgemäß sind solche Zusammensetzungen bevorzugt, die die folgenden Komponenten enthalten:
a) 1 bis 60 Gew.-%, insbesondere 5 bis 40 Gew.-% mit Gruppen der Formel (I) oberflächenfunktionalisierten Füllstoff,
b) 0 bis 40 Gew.-%, insbesondere 0 bis 30 Gew.-% Füllstoff, der nicht mit Gruppen der Formel (I) oberflächenfunktionalisiert ist,
c) 0 bis 70 Gew.-%, insbesondere 1 bis 40 Gew.-% Verdünnerund/oder Vernetzermonomer,
d) 0,1 bis 5,0 Gew.-%, insbesondere 0,2 bis 2,0 Gew.-%, besonders bevorzugt 0,2 bis 1,0 Gew.-% Initiator für die radikalische Polymerisation,
e) 0 bis 70 Gew.-%, insbesondere 0 bis 50 Gew.-% acides Monomer und/oder Lösungsmittel.

Alle Prozentangaben beziehen sich auf die Gesamtmasse der Zusammensetzung. Ganz besonders bevorzugt sind Zusammensetzungen, die mindestens ein acides Monomer oder mindestens ein Vernetzermonomer, insbesondere mindestens ein acides Monomer und mindestens ein Vernetzermonomer oder mindestens ein acides Vernetzermonomer enthalten.

Die erfindungsgemäßen Zusammensetzungen eignen sich besonders als Dentalmaterialien, insbesondere als Adhäsive, Zemente, vorzugsweise selbsthaftende Zemente wie z.B. Befestigungszemente, und Komposite, vorzugsweise Füllungskomposite. Solche Dentalmaterialien zeichnen sich durch eine sehr gute Haftung auf der Zahnhartsubstanz, d.h. auf Schmelz und Dentin, aus.

Die bevorzugten erfindungsgemäßen Zusammensetzungen härten unter Ausbildung von stark vernetzten Polymernetzwerken aus, die in Wasser wenig oder gar nicht quellen.

Die Erfindung betrifft ferner auch ein Verfahren zur Herstellung einer erfindungsgemäßen Zusammensetzung, bei dem der Füllstoff mit mindestens einem Silan umgesetzt wird und der erhaltene oberflächenfunktionalisierte Füllstoff mit den weiteren Bestandteilen der Zusammensetzung gemischt wird. Bevorzugte Ausführungsformen der Umsetzung des Füllstoffs mit mindestens einem Silan sind wie oben beschrieben.

Die Erfindung betrifft schließlich auch die Verwendung eines mit Gruppen der Formel (I) oberflächenfunktionalisierten Füllstoffs zur Herstellung eines Dentalmaterials, insbesondere eines Adhäsivs oder Zements.

Die Erfindung wird im Folgenden anhand von Beispielen näher erläutert.

### Beispiele:

### Beispiel 1:

### Oberflächenfunktionalisierung von SiO₂-Nanopartikeln (d = 13 nm) eines SiO₂-Organosols mit Aldehydgruppen

### 1. Stufe: Funktionalisierung von SiO₂-Nanopartikeln mit SH-Gruppen

2,12 g 3-Mercaptopropyltrimethoxysilan (10,8 mmol) wurden zu 60,0 g SiO₂-Organosol (NanO G 502-31, Clariant; 30 Gew.-% SiO₂ in Isopropanol) gegeben. Anschließend wurden 0,584 g (32,4 mmol bezogen auf Wasser) einer 0,5 N HCl-Lösung zugegeben und 48 h bei Raumtemperatur gerührt. Dann wurde die Dispersion auf 0 °C gekühlt und 0,98 g (9,0 mmol) Chlortrimethylsilan zugetropft. Nach 24 h Rühren bei Raumtemperatur wurden 40 ml Toluol zugegeben und bei 40 °C und 140 mbar - 80 mbar wurden das Isopropanol und das entstandene Methanol abdestilliert. Das dabei mit abdestillierende Toluol wurde anschlieβend wieder zugegeben, so dass die Konzentration an SiO₂ in der Dispersion nicht über 30,0 Gew.-% stieg. Es wurden 75,3 g einer leicht viskosen, trüben und thixotropen Flüssigkeit erhalten.

### 2. Stufe: Thiol-En-Addition von Acrolein an SHfunktionalisierte SiO₂-Nanopartikel

Unter Argon wurden 0,605 g (10,8 mmol) Acrolein sowie eine Spatelspitze Hydrochinon im Kolben vorgelegt. Bei 0 bis 5 °C wurden dann 75,3 g des in der 1. Stufe hergestellten Organosols innerhalb von 11 h zugetropft. Das Eisbad wurde entfernt und die Dispersion wurde 1 Woche bei Raumtemperatur gerührt. In dem erhaltenen Organosol war kein Acrolein mehr vorhanden. Das Produkt enthielt etwa 58 % Aldehydgruppen, bezogen auf das eingesetzte Trialkoxysilan. Der Glührückstand des leicht viskosen, trüben und thixotropen Organosols betrug 7,1 % SiO₂.

### Beispiel 2:

### Herstellung eines mit Aldehydgruppen oberflächenfunktionalisierten SiO₂-Organosols mit einem radikalisch vernetzbaren Dispersionsmittel

25,0 g N,N'-Diethyl-1,3-bis(acrylamido)-propan wurden in 70,7 g des mit Aldehydgruppen funktionalisierten SiO₂-Organosols aus der 2. Stufe von Beispiel 1 (SiO₂-Gehalt: 5 g) gelöst. Es entstand eine transluzente Lösung. Anschließend wurde das Toluol am Rotationsverdampfer bei 40 °C entfernt. Es wurde eine leicht bräunliche, transluzent-trübe Flüssigkeit mit einer Viskosität von 7,0-5,5 Pa·s (1-100 s⁻¹) und einem Glührückstand von 15,8 % erhalten, die sich nach Zugabe eines radikalischen Initiators zu einem unlöslichen Feststoff aushärten ließ.

### Beispiel 3:

### Oberflächenfunktionalisierung von pyrogener Kieselsäure mit Aldehydgruppen

### 1. Stufe: Funktionalisierung von pyrogener Kieselsäure OX-50 mit SH-Gruppen

20,0 g pyrogene Kieselsäure OX-50 wurden in 200 g Ethanol suspendiert. Dann wurden 0,56 g einer 0,5 N HCl-Lösung zugegeben und die Suspension auf 70 °C erwärmt. Es wurden 2,04 g (10,4 mmol) 3-Mercaptopropyltrimethoxysilan zugegeben, und die Suspension wurde 30 h bei 70 °C gerührt. Anschließend wurde das Lösungsmittel bei 40 °C am Rotationsverdampfer entfernt. Das erhaltene weiße Pulver wurde in 40 g Aceton dispergiert und dann durch Zentrifugation wieder abgetrennt. Anschließend wurde das Pulver in 50 g Ethanol dispergiert, abzentrifugiert, zuletzt in Cyclohexan dispergiert und wieder abzentrifugiert. Das erhaltene Pulver wurde am Rotationsverdampfer bei 8·10⁻² mbar getrocknet. Die Belegung mit Mercaptogruppen betrug 0,12 mmol/g SiO₂ und wurde über den Schwefel-Gehalt (0,38 Gew.-%, Elementaranalyse) der Probe bestimmt.

### 2. Stufe: Thiol-En-Addition von Acrolein an SHfunktionalisierte SiO₂-Partikel

10,0 g funktionalisierte Partikel aus der 1. Stufe wurden in 30 g Toluol im Ultraschall dispergiert. Anschließend wurden 0,24 g (4,3 mmol) Acrolein zugetropft und die Suspension 48 h bei 50 °C gerührt. Dann wurden das Lösungsmittel und nicht abreagiertes Acrolein bei 40 °C am Rotationsverdampfer entfernt und das Pulver bei 8·10⁻² mbar getrocknet. Die Belegung mit funktionalisiertem Silan betrug 0,10 mmol/g SiO₂ und wurde über den Schwefel-Gehalt (0,33 Gew.-%, Elementaranalyse) der Probe bestimmt.

### Beispiel 4:

### Oberflächenfunktionalisierung von pyrogener Kieselsäure (Aerosil 200) mit Phosphonsäuregruppen

### 1. Stufe: Funktionalisierung von pyrogener Kieselsäure mit Diethylphosphonat-Gruppen

25,0 g Aerosil 200 wurden in 750 g Cyclohexan suspendiert. Dann wurden 13,6 g (41,4 mmol) Diethylphosphorylethyltriethoxysilan und 3,68 g (62,25 mmol) n-Propylamin zugegeben. Das Gemisch wurde 30 h bei 70 °C gerührt. Das Lösungsmittel wurde bei 40 °C am Rotationsverdampfer entfernt und das Produkt noch 3 Tage bei 50 °C im Trockenschrank getrocknet. Das Pulver wurde dann zum Waschen in 150 ml Ethanol suspendiert und mittels Druckfiltration (0,45 µm) vom Lösungsmittel abgetrennt. Das Pulver wurde noch einmal analog mit Ethanol und dann einmal mit Cyclohexan gewaschen. Es wurde anschließend nochmals 3 Tage bei 50 °C im Trockenschrank getrocknet. Der Glührückstand betrug 93,1 Gew.-%. Die Belegung mit funktionalisiertem Silan betrug 0,66 mmol/g SiO₂ und wurde über den Phosphor-Gehalt (1,78 Gew.-%, Elementaranalyse) der Probe bestimmt.

### 2. Stufe: Freisetzung der Phosphonsäuregruppen

25,0 g silanisiertes Aerosil 200 aus der 1. Stufe wurden in 460 g Salzsäure (32 Gew.-%) suspendiert und 46 h unter Rückfluss erhitzt. Dann wurde die Salzsäurelösung im Vakuum bei 40 °C entfernt und das Pulver 3 Tage im Trockenschrank bei 50 °C getrocknet. Die modifizierten Partikel wurden dann in 150 ml Wasser redispergiert und mittels Druckfiltration (0,45 µm) filtriert. Der Vorgang wurde noch zweimal wiederholt und das Pulver anschließend 3 Tage im Trockenschrank bei 50 °C getrocknet. Der Glührückstand betrug 96,0 Gew.-%. Die Belegung mit funktionalisiertem Silan betrug 0,58 mmol/g SiO₂ und wurde über den Phosphor-Gehalt (1,85 Gew.-%, Elementaranalyse) der Probe bestimmt.

### Beispiel 5:

### Zusätzliche Oberflächenfunktionalisierung von mit Phosphonsäuregruppen modifizierter pyrogener Kieselsäure mit Methacrylatgruppen

11,0 g modifiziertes Aerosil aus der 2. Stufe von Beispiel 4 wurden in 330 g n-Hexan suspendiert. Anschließend wurden 1,99 g (9,0 mmol) 3-Methacryloyloxypropyldimethylchlorsilan (ABCR), gelöst in 30 ml n-Hexan, langsam zugetropft. Das Gemisch wurde noch 47 h bei Raumtemperatur gerührt. Dann wurde das Lösungsmittel im Vakuum bei 40 °C entfernt und das Pulver 3 Tage im Trockenschrank bei 50 °C getrocknet. Die Partikel wurden in 150 ml Ethanol dispergiert und mittels Druckfiltration (0,45 µm) filtriert, ein zweites Mal in Ethanol dispergiert und filtriert und schließlich noch einmal in 150 ml Cyclohexan dispergiert und abfiltriert. Das Pulver wurde 3 Tage im Trockenschrank bei 50 °C getrocknet. Der Glührückstand betrug 94,8 Gew.-%. IR-spektroskopisch konnte durch das Auftreten einer neuen Bande bei 1636 cm⁻¹ das Vorhandensein von Methacrylatgruppen nachgewiesen werden.

### Beispiel 6:

### Oberflächenfunktionalisierung von pyrogener Kieselsäure (Aerosil OX-50) mit Phosphonsäuregruppen

### 1. Stufe: Funktionalisierung von pyrogener Kieselsäure mit Diethylphosphonat-Gruppen

20,0 g pyrogene Kieselsäure OX-50 wurden in 200 g Ethanol suspendiert. Dann wurden zunächst 3,41 g (10,4 mmol) Diethylphosphorylethyltriethoxysilan und anschließend 0,56 g einer 0,5 N Salzsäure-Lösung zugegeben. Die Suspension wurde auf 70 °C erwärmt und 30 h bei dieser Temperatur gerührt. Dann wurden die flüchtigen Bestandteile bei 40 °C am Rotationsverdampfer entfernt und das Pulver 3 Tage bei 50 °C im Trockenschrank getrocknet. Das Pulver wurde zum Waschen in 50 ml Ethanol suspendiert und mittels Zentrifugation (bis 5000 U/min) vom Lösungsmittel abgetrennt. Das Pulver wurde noch einmal analog mit Ethanol und dann einmal mit 50 ml Cyclohexan gewaschen. Es wird anschließend nochmals 3 Tage bei 50 °C im Trockenschrank getrocknet. Die Belegung mit funktionalisiertem Silan betrug 0,11 mmol/g SiO₂ und wurde über den Phosphor-Gehalt (0,32 Gew.-%, Elementaranalyse) der Probe bestimmt.

### 2. Stufe: Freisetzung der Phosphonsäuregruppen

10,0 g modifiziertes OX-50 aus der 1. Stufe wurden in 100 g Salzsäure (32 Gew.-%) dispergiert und 24 h bei 100 °C unter Rückfluss erhitzt. Dann wurde die Salzsäurelösung am Rotationsverdampfer bei 40 °C im Vakuum entfernt. Das Pulver wurde anschließend 3 Tage bei 50 °C im Trockenschrank getrocknet. Die modifizierten Partikel wurden dann in 50 ml Wasser redispergiert und mittels Zentrifugation wieder abgetrennt. Der Vorgang wurde noch zweimal wiederholt und das Pulver anschließend 3 Tage im Trockenschrank bei 50 °C getrocknet. Die Belegung mit funktionalisiertem Silan betrug 0,12 mmol/g SiO₂ und wurde über den Phosphor-Gehalt (0,36 Gew.-%, Elementaranalyse) der Probe bestimmt.

### Beispiel 7:

### Schmelz-Dentin-Adhäsiv, das aldehydfunktionalisierte SiO₂-Partikel enthält

Zur Untersuchung der Schmelz- und Dentinhaftung wurden die Adhäsive A (erfindungsgemäß) und B (Vergleichsbeispiel) durch Mischen der Ausgangskomponenten mit der in nachfolgender Tabelle dargestellten Zusammensetzung (Angabe in Gew.-%) hergestellt. Anschließend wurde die Haftung der Adhäsive auf Zahnschmelz und Dentin ermittelt.

Dazu wurden Rinderzähne so in Kunststoffzylinder eingebettet, dass sich das Dentin bzw. der Zahnschmelz und der Kunststoff in einer Ebene befanden. Nach Anschleifen der Prüfkörper wurde mit einem Microbrush eine Schicht Adhäsiv obiger Formulierung 30 s auf die Dentinoberfläche einmassiert, mit einem Luftbläser leicht verblasen und mit einer Photopolymerisationslampe Astralis 7 (Ivoclar Vivadent AG) für 20 s belichtet. Dann wurde auf die Adhäsivschicht das Füllungskomposit Tetric® Ceram (Icoclar Vivadent AG) aufgebracht und 40 s mit der Lampe Astralis 7 ausgehärtet. Anschließend wurden die Prüfkörper 24 h bei 37 °C in Wasser gelagert und die Scherhaftfestigkeit gemäß der ISO-Richtlinie "ISO 1994-ISO TR 11405: Dental Materials Guidance on Testing of Adhesion to Tooth Structure" bestimmt. Die Ergebnisse belegen, dass die aldehydfunktionalisierten Partikel zu einer Verbesserung der Scherhaftfestigkeit auf Dentin und Zahnschmelz führen.

| **Komponenten/Haftwerte** | **Adhäsiv A** (erfindungsgemäß) | **Adhäsiv B** (Vergleich) |
|---|---|---|
| Bismethacrylamidphosphat¹⁾ | 14,6 | 14,6 |
| 2-(Acryloylamino)-bernsteinsäure | 9,7 | 9,7 |
| Wasser | 23,0 | 23,0 |
| Aerosil 200, unmodifiziert | - | 3 |
| SiO₂-Partikel aus Beispiel 2²⁾ | 2,4 | - |
| N,N'-Diethyl-1,3-bis(acrylamido)-propan³⁾ | 47,6 | 47,0 |
| N-(5-Hydroxypentyl)methacrylamid | 2,0 | 2,0 |
| Photoinitiator⁴⁾ | 0,7 | 0,7 |
| Schmelzhaftung (MPa) | 21 | 16 |
| Dentinhaftung (MPa) | 23 | 21 |

| | | |
|---|---|---|
| ¹) 1,3-Bis-(N-methacryloylamino)-propan-2-yl-dihydrogenphosphat ²) als Organosol eingearbeitet, Angabe ohne das im Organosol enthaltene N,N'-Diethyl-1,3-bis(acrylamido)-propan ³) bei Adhäsiv A einschließlich der im verwendeten Organosol enthaltenen Menge ⁴) Photoinitiator: 0,3 Gew.-% Campherchinon, 0,4 Gew.-% 4-Dimethylaminobenzoesäureethylester | | |

## Patentansprüche

1. Polymerisierbare Zusammensetzung, **dadurch gekennzeichnet, dass** sie
mindestens einen mit Gruppen der Formel (I) oberflächenfunktionalisierten Füllstoff, bei dem Gruppen der Formel (I)
(A)ₐ-Z-Y-R²-SiR¹₃₋ₘ-(O---)ₘ (I),
in der
R¹ für C₁-C₁₅-Alkyl, C₂-C₅-Alkenyl oder Phenyl steht,
R² entfällt oder für unverzweigtes oder verzweigtes C₁- C₆-Alkylen steht,
Y entfällt oder für eine Ether-, Thioether-, Amid-, Es- ter- oder Urethangruppe steht,
Z entfällt oder für einen mindestens zweiwertigen line- aren oder verzweigten aliphatischen Rest mit 2 bis 40 Kohlenstoffatomen, der durch eine oder mehrere Ether-, Thioether-, Amid- oder Estergruppen unterbro- chen sein kann und eine oder mehrere cycloaliphati- sche Gruppen mit mindestens 3 Kohlenstoffatomen und/oder eine oder mehrere aromatische Gruppen mit mindestens 6 Kohlenstoffatomen enthalten kann, einen mindestens zweiwertigen cycloaliphatischen Rest mit mindestens 3 Kohlenstoffatomen oder einen mindestens zweiwertigen aromatischen Rest mit mindestens 6 Koh- lenstoffatomen steht,
A jeweils unabhängig für -COOH, -P(O)(OH)₂, -O- P(O)(OH)₂, -SO₂OH, -C(O)-O-C(O)-, -CHO, -NH-C(O)-CHO, -C(O)-CHO, -C(O)-CH₂-C(O)-CH₃, -N=C=O oder -O-C(O)- CH₂-C(O)-CH₃ steht,
a 1 bis 6 ist und
m 1 bis 3 ist,
wobei R² und Z nicht beide entfallen können,
wobei R² und Z jeweils nur entfallen können, wenn zugleich auch Y entfällt, und
wobei a 1 ist, wenn Z entfällt,
über mindestens ein an das Siliciumatom der Gruppe der Formel (I) gebundenes Sauerstoffatom mit dem Füllstoff verknüpft sind, und
einen Initiator für die radikalische Polymerisation enthält.

2. Polymerisierbare Zusammensetzung nach Anspruch 1, bei der
R¹ für C₁-C₆-Alkyl oder Phenyl steht,
R² für lineares oder verzweigtes C₁-C₃-Alkylen steht,
Y entfällt oder für eine Ether-, Thioether-, Ester oder Urethangruppe steht,
Z entfällt oder für einen mindestens zweiwertigen line- aren oder verzweigten aliphatischen Rest mit 2 bis 20 Kohlenstoffatomen, der durch eine oder mehrere Ether-, Thioether-, Amid- oder Estergruppen unterbro- chen sein kann und eine oder mehrere cycloaliphati- sche Gruppen mit mindestens 3 Kohlenstoffatomen und/oder eine oder mehrere aromatische Gruppen mit mindestens 6 Kohlenstoffatomen enthalten kann, einen mindestens zweiwertigen cycloaliphatischen Rest mit mindestens 3 Kohlenstoffatomen oder einen mindestens zweiwertigen aromatischen Rest mit mindestens 6 Koh- lenstoffatomen steht,
A jeweils unabhängig für -COOH, -P(O)(OH)₂, -O- P(O)(OH)₂, -SO₂OH, -CHO, -NH-C(O)-CHO oder -O-C(O)- CH₂-C(O)-CH₃ steht,
a 1 bis 3 ist und
m 1 bis 3 ist.

3. Polymerisierbare Zusammensetzung nach Anspruch 1 oder 2, **dadurch gekennzeichnet, dass** der Füllstoff ein partikulärer Füllstoff mit einer mittleren Partikelgröße von 1 nm bis 10 µm ist.

4. Polymerisierbare Zusammensetzung nach einem der Ansprüche 1 bis 3, **dadurch gekennzeichnet, dass** der Füllstoff monodisperser, nanopartikulärer Füllstoff auf Basis von SiO₂, Oxiden der Elemente Zr, Ti, Al, Y, La, Ce und/oder Yb oder deren Mischoxiden mit SiO₂ ist.

5. Polymerisierbare Zusammensetzung nach einem der Ansprüche 1 bis 4, **dadurch gekennzeichnet, dass** der Füllstoff eine mittlere Partikelgröße von 5 bis 200 nm aufweist.

6. Polymerisierbare Zusammensetzung nach Anspruch 5, **dadurch gekennzeichnet, dass** der Füllstoff eine mittlere Partikelgröße von 10 bis 100 nm aufweist.

7. Polymerisierbare Zusammensetzung nach Anspruch 6, **dadurch gekennzeichnet, dass** der Füllstoff eine mittlere Partikelgröße von 10 bis 50 nm aufweist.

8. Polymerisierbare Zusammensetzung nach einem der Ansprüche 1 bis 7, **dadurch gekennzeichnet, dass** der mit Gruppen der Formel (I) oberflächenfunktionalisierte Füllstoff mindestens 0,01 mmol Gruppen der Formel (I) pro Gramm des Füllstoffs enthält.

9. Polymerisierbare Zusammensetzung nach Anspruch 8, **dadurch gekennzeichnet, dass** der mit Gruppen der Formel (I) oberflächenfunktionalisierte Füllstoff 0,05 - 2 mmol Gruppen der Formel (I) pro Gramm des Füllstoffs enthält.

10. Polymerisierbare Zusammensetzung nach Anspruch 9, **dadurch gekennzeichnet, dass** der mit Gruppen der Formel (I) oberflächenfunktionalisierte Füllstoff 0,1 bis 1 mmol Gruppen der Formel (I) pro Gramm des Füllstoffs enthält.

11. Polymerisierbare Zusammensetzung nach einem der Ansprüche 1 bis 10, **dadurch gekennzeichnet, dass** der Füllstoff zusätzlich mit mindestens einer weiteren Gruppe oberflächenmodifiziert ist.

12. Polymerisierbare Zusammensetzung nach einem der Ansprüche 1 bis 11, **dadurch gekennzeichnet, dass** sie mindestens ein radikalisch polymerisierbares Monomer enthält.

13. Zusammensetzung nach einem der Ansprüche 1 bis 12, **dadurch gekennzeichnet, dass** sie mindestens ein Monomer mit 2 oder mehr polymerisierbaren Gruppen und/oder mindestens ein Monomer mit einer oder mehreren aciden Gruppen enthält.

14. Zusammensetzung nach einem der Ansprüche 1 bis 13, **dadurch gekennzeichnet, dass** sie einen Initiator für die Photopolymerisation enthält.

15. Zusammensetzung nach einem der Ansprüche 1 bis 14, **dadurch gekennzeichnet, dass** sie ferner einen Füllstoff enthält, der nicht mit Gruppen der Formel (I) oberflächenfunktionalisiert ist.

16. Zusammensetzung nach einem der Ansprüche 1 bis 15, **dadurch gekennzeichnet, dass** sie
a) 1 bis 60 Gew.-% mit Gruppen der Formel (I) oberflächenfunktionalisierten Füllstoff,
b) 0 bis 40 Gew.-% Füllstoff, der nicht mit Gruppen der Formel (I) oberflächenfunktionalisiert ist,
c) 1 bis 70 Gew.-% Verdünner- und/oder Vernetzermonomer,
d) 0,1 bis 5,0 Gew.-% Initiator für die radikalische Polymerisation,
e) 0 bis 70 Gew.-% acides Monomer und/oder Lösungsmittel enthält.

17. Verfahren zur Herstellung einer Zusammensetzung gemäß einem der Ansprüche 1 bis 16, bei dem der Füllstoff mit mindestens einem Silan umgesetzt wird und der erhaltene oberflächenfunktionalisierte Füllstoff mit den weiteren Bestandteilen der Zusammensetzung gemischt wird.

18. Verfahren nach Anspruch 17, bei der das Silan die Formel (II) aufweist
Aₐ-Z-Y-R²SiXₙR¹₃₋ₙ (II),
in der
X für Halogen, Hydroxy, C₁-C₅-Alkoxy oder C₁-C₃-Acyloxy steht,
n 1 bis 3 ist, und
R¹, R², Y, Z und A wie in einem der Ansprüche 1 bis 16 de- finiert sind.

19. Verfahren nach Anspruch 17 oder 18, bei dem
a) das Silan in flüssiger Form mit dem Füllstoff vermischt wird und
b) der Füllstoff zur Abtrennung von Kondensationsprodukten getrocknet wird.

20. Verfahren nach Anspruch 17 oder 18, bei dem
a) der Füllstoff in einer Lösung des Silans in einem Lösungsmittel dispergiert wird und
b) der Füllstoff abgetrennt und gegebenenfalls einmal oder mehrmals mit dem Lösungsmittel aus Schritt (a) und/oder mindestens einem anderen Lösungsmittel gewaschen wird,
c) gegebenenfalls der Füllstoff einer Wärmebehandlung unterzogen und gegebenenfalls nochmals gewaschen wird,
d) der Füllstoff getrocknet wird und
e) gegebenenfalls der Füllstoff gemahlen wird.

21. Verwendung eines oberflächenfunktionalisierten Füllstoffs, bei dem Gruppen der Formel (I)
Aₐ-Z-Y-R²SiXₙR¹₃₋ₙ-(O---)₃ (I),
in der
R¹ für C₁-C₁₅-Alkyl, C₂-C₅-Alkenyl oder Phenyl steht,
R² entfällt oder für unverzweigtes oder verzweigtes C₁- C₆-Alkylen steht,
Y entfällt oder für eine Ether-, Thioether-, Amid-, Es- ter- oder Urethangruppe steht,
Z entfällt oder für einen mindestens zweiwertigen line- aren oder verzweigten aliphatischen Rest mit 2 bis 40 Kohlenstoffatomen, der durch eine oder mehrere Ether-, Thioether-, Amid- oder Estergruppen unterbro- chen sein kann und eine oder mehrere cycloaliphati- sche Gruppen mit mindestens 3 Kohlenstoffatomen und/oder eine oder mehrere aromatische Gruppen mit mindestens 6 Kohlenstoffatomen enthalten kann, einen mindestens zweiwertigen cycloaliphatischen Rest mit mindestens 3 Kohlenstoffatomen oder einen mindestens zweiwertigen aromatischen Rest mit mindestens 6 Koh- lenstoffatomen steht,
A jeweils unabhängig für -COOH, -P(O)(OH)₂, -O- P(O) (OH)₂, -SO₂OH, -C(O)-O-C(O)-, -CHO, -NH-C(O)-CHO, -C(O)-CHO, -C(O)-CH₂-C(O)-CH₃, -N=C=O oder -O-C(O)- CH₂-C(O)-CH₃ steht,
a 1 bis 6 ist und
m 1 bis 3 ist,
wobei R² und Z nicht beide entfallen können,
wobei R² und Z jeweils nur entfallen können, wenn zugleich auch Y entfällt, und
wobei a 1 ist, wenn Z entfällt,
über mindestens ein an das Siliciumatom der Gruppe der Formel (I) gebundenes Sauerstoffatom mit dem Füllstoff verknüpft sind,
zur Herstellung eines Dentalmaterials.

22. Verwendung nach Anspruch 21, zur Herstellung eines Adhäsivs oder Zements.

23. Verwendung nach Anspruch 21 oder 22, wobei der Füllstoff nach einem Verfahren erhältlich ist, bei dem der Füllstoff mit mindestens einem Silan umgesetzt wird.

## Claims

1. A polymerisable composition, comprising at least one filler that is surface modified with groups of Formula (I),
(A)ₐ-Z-Y-R²-SiR¹₃₋ₘ-(O---)ₘ (I),
in which
R¹ stands for C₁-C₁₅ alkyl, C₂-C₅ alkenyl or phenyl,
R² is absent or stands for unbranched or branched C₁-C₆ alkylene,
Y is absent or stands for an ether, thioether, amide, ester or urethane group,
Z is absent or stands for an at least divalent linear or branched alkyl group containing 2 to 40 carbon atoms which can be interrupted by one or more ether, thioether, amide or ester groups and can comprise one or more cycloaliphatic groups containing at least 3 carbon atoms and/or one or more aromatic groups containing at least 6 carbon atoms, an at least divalent cycloaliphatic group containing at least 3 carbon atoms or an at least divalent aromatic group containing at least 6 carbon atoms,
A each independently stands for -COOH, -P(O)(OH)₂, -O-P(O)(OH)₂, -SO₂OH, -C(O)-O-C(O)-, -CHO, -NH-C(O)-CHO, -C(O)-CHO, -C(O)-CH₂-C(O)-CH₃, -N=C=O or -O-C(O)-CH₂-C(O)-CH₃
a is 1 to 6 and
m is 1 to 3,
wherein R² and Z cannot both be absent,
wherein R² and Z can each be absent only if Y is also absent as well, and
wherein a is 1, if Z is absent,
in which groups of Formula (I) are bonded to the filler through at least one oxygen atom that is bound to the silicon atom of the group of Formula (I), and
said composition comprises an initiator for the radical polymerisation.

2. The polymerisable composition according to claim 1, in which
R¹ stands for C₁-C₆ alkyl or phenyl,
R² stands for linear or branched C₁-C₃ alkylene,
Y is absent or stands for an ether, thioether, ester or urethane group,
Z is absent or stands for an at least divalent linear or branched alkyl group containing 2 to 20 carbon atoms which can be interrupted by one or more ether, thioether, amide or ester groups and can comprise one or more cycloaliphatic groups containing at least 3 carbon atoms and/or one or more aromatic groups containing at least 6 carbon atoms; an at least divalent cycloaliphatic group containing at least 3 carbon atoms or an at least divalent aromatic group containing at least 6 carbon atoms,
A each stands independently for -COOH, -P(O)(OH)₂, -O- P(O)(OH)₂, -SO₂OH, -CHO, -NH-C(O)-CHO or -O-C(O)-CH₂-C(O)-CH₃,
a is 1 to 3 and
m is 1 to 3.

3. The polymerisable composition according to claim 1 or 2, **characterised in that** the filler is a particulate filler with a mean particle size of 1 nm to 10 µm.

4. The polymerisable composition according to one of claims 1 to 3, **characterised in that** the filler is a monodisperse, nanoparticulate filler based on SiO₂, oxides of the element Zr, Ti, Al, Y, La, Ce and/or Yb or their mixed oxides with SiO₂.

5. The polymerisable composition according to one of claims 1 to 4, **characterised in that** the filler has a mean particle size of 5 nm to 200 nm.

6. The polymerisable composition according to claim 5, **characterised in that** the filler has a mean particle size of 10 nm to 100 nm.

7. The polymerisable composition according to claim 6, **characterised in that** the filler has a mean particle size of 10 nm to 50 nm.

8. The polymerisable composition according to one of claims 1 to 7, **characterised in that** the filler that is surface functionalised with groups of Formula (I) comprises at least 0.01 mmol of groups of Formula (I) per gram of the filler.

9. The polymerisable composition according to claim 8, **characterised in that** the filler that is surface functionalised with groups of Formula (I) comprises 0.05 - 2 mmol of groups of Formula (I) per gram of the filler.

10. The polymerisable composition according to claim 9, **characterised in that** the filler that is surface functionalised with groups of Formula (I) comprise 0.1 to 1 mmol of groups of Formula (I) per gram of the filler.

11. The polymerisable composition according to one of claims 1 to 10, **characterised in that** the filler is further surface modified with at least one additional group.

12. The polymerisable composition according to one of claims 1 to 11, comprising at least one radically polymerisable monomer.

13. The composition according to one of claims 1 to 12, comprising at least one monomer containing 2 or more polymerisable groups and/or at least one monomer containing one or more acidic groups.

14. The composition according to one of claims 1 to 13, additionally comprising an initiator for photopolymerisation.

15. The composition according to one of claims 1 to 14, further comprising a filler that is not surface functionalised by groups of Formula (I).

16. The composition according to one of claims 1 to 15, comprising
a) 1 to 60 wt.% of filler that is surface functionalised with groups of Formula (I)
b) 0 to 40 wt.% filler that is not surface functionalised with groups of Formula (I)
c) 1 to 70 wt.% diluent monomer and/or crosslinking monomer,
d) 0.1 to 5.0 wt.% initiator for the radical polymerisation,
e) 0 to 70 wt.% acidic monomer and/or solvent.

17. A process for manufacturing a composition according to one of claims 1 to 16, in which process the filler is treated with at least one silane and the resulting surface functionalised filler is then mixed with the further ingredients of the composition.

18. The process according to claim 17, in which process the silane has the Formula (II)
(A)ₐ-Z-Y-R²_SiXₙR¹₃₋ₙ (II),
in which
X stands for halogen, hydroxy, C₁-C₅ alkoxy or C₁-C₃ acyloxy,
n is 1 to 3, and
R¹, R², Y, Z and A are defined as in one of claims 1 to 16.

19. The process according to claim 17 or 18, in which process
a) the silane is mixed in liquid form with the filler and
b) the filler is dried in order to remove condensation products.

20. The process according to claim 17 or 18, in which process
a) the filler is dispersed in a solution of the silane in a solvent and
b) the filler is separated and optionally washed once or a plurality of times with the solvent of step (a) and/or with at least one other solvent,
c) the filler is optionally subjected to a thermal treatment and optionally washed again,
d) the filler is dried and
e) the filler is optionally ground.

21. Use of a surface functionalised filler, in which groups of Formula (I)
(A)ₐ-Z-Y-R²-SiR¹₃₋ₘ-(O---)ₘ (I),
in which
R¹ stands for C₁-C₁₅ alkyl, C₂-C₅ alkenyl or phenyl,
R² is absent or stands for unbranched or branched C₁-C₆ alkylene,
Y is absent or stands for an ether, thioether, amide, ester or urethane group,
Z is absent or stands for an at least divalent linear or branched alkyl group containing 2 to 40 carbon atoms which can be interrupted by one or more ether, thioether, amide or ester groups and can comprise one or more cycloaliphatic groups containing at least 3 carbon atoms and/or one or more aromatic groups containing at least 6 carbon atoms, an at least divalent cycloaliphatic group containing at least 3 carbon atoms or an at least divalent aromatic group containing at least 6 carbon atoms,
A each independently stands for -COOH, -P(O)(OH)₂, -O-P(O)(OH)₂, -SO₂OH, -C(O)-O-C(O)-, -CHO, -NH-C(O)-CHO, -C(O)-CHO, -C(O)-CH₂-C(O)-CH₃, -N=C=O or-O-C(O)-CH₂-C(O)-CH₃,
a is 1 to 6 and
m is 1 to 3,
wherein R² and Z cannot both be absent,
wherein R² and Z can each be absent only if Y is also absent as well, and
wherein a is 1 if Z is absent,
are bonded to the filler through at least one oxygen atom that is bound to the silicon atom of the group of Formula (I)
for the manufacture of a dental material.

22. Use according to claim 21 for the manufacture of an adhesive or cement.

23. Use according to claim 21 or 22, wherein the filler is obtainable according to a process, in which the filler is treated with at least one silane.

## Revendications

1. Composition polymérisable, **caractérisée en ce qu'**elle comprend :
au moins une charge fonctionnalisée en surface avec des groupes de formule (I), dans laquelle des groupes de formule (I) :
(A)ₐ-Z-Y-R²-SiR¹₃₋ₘ-(O---)ₘ (I)
dans laquelle
- R¹ représente un groupe alkyle en C₁₋₁₅, alcényle en C₂₋₅ ou phényle,
- R² ne représente rien ou représente un groupe alcanediyle en C₁₋₆, linéaire ou ramifié,
- Y ne représente rien ou représente un groupe ou chaînon de type éther, thioéther, amide, ester ou uréthane,
- Z ne représente rien ou représente
un groupe aliphatique au moins divalent, linéaire ou ramifié, comportant de 2 à 40 atomes de carbone, qui peut être interrompu par un ou plusieurs groupe(s) ou chaînon(s) de type éther, thioéther, amide ou ester et contenir un ou plusieurs groupe(s) cycloaliphatique(s) comportant au moins trois atomes de carbone et/ou un ou plusieurs groupe(s) aromatique(s) comportant au moins six atomes de carbone,
un groupe cycloaliphatique au moins divalent, comportant au moins 3 atomes de carbone,
ou un groupe aromatique au moins divalent, comportant au moins 6 atomes de carbone,
- chaque symbole A représente indépendamment une entité de formule -COOH, -P(O)(OH)₂, -O-P(O)(OH)₂, -SO₂OH, -CHO, -C(O)-O-C(O)-, -NH-C(O)-CHO, -C(O)-CHO, -N=C=O, -C(O)-CH₂-C(O)-CH₃, ou -O-C(O)-CH₂-C(O)-CH₃,
- l'indice a vaut de 1 à 6,
- et l'indice m vaut de 1 à 3,
étant entendu :
- que les symboles R² et Z ne peuvent pas tous les deux à la fois ne rien représenter,
- que chacun des symboles R² et Z ne peut ne rien représenter que si à la fois Y aussi ne représente rien,
- et que si Z ne représente rien, l'indice a vaut 1,
sont liés à la charge par l'intermédiaire d'au moins un atome d'oxygène lié à l'atome de silicium du groupe de formule (I),
et un amorceur de polymérisation radicalaire.

2. Composition polymérisable conforme à la revendication 1, dans laquelle :
- R¹ représente un groupe alkyle en C₁₋₆ ou phényle,
- R² représente un groupe alcanediyle en C₁₋₃, linéaire ou ramifié,
- Y ne représente rien ou représente un groupe ou chaînon de type éther, thioéther, ester ou uréthane,
- Z ne représente rien ou représente
un groupe aliphatique au moins divalent, linéaire ou ramifié, comportant de 2 à 20 atomes de carbone, qui peut être interrompu par un ou plusieurs groupe(s) ou chaînon(s) de type éther, thioéther, amide ou ester et contenir un ou plusieurs groupe(s) cycloaliphatique(s) comportant au moins trois atomes de carbone et/ou un ou plusieurs groupe(s) aromatique(s) comportant au moins six atomes de carbone,
un groupe cycloaliphatique au moins divalent, comportant au moins 3 atomes de carbone,
ou un groupe aromatique au moins divalent, comportant au moins 6 atomes de carbone,
- chaque symbole A représente indépendamment une entité de formule -COOH, -P(O)(OH)₂, -O-P(O)(OH)₂, -SO₂OH, -NH-C(O)-CHO, -CHO, ou -O-C(O)-CH₂-C(O)-CH₃,
- l'indice a vaut de 1 à 3,
- et l'indice m vaut de 1 à 3.

3. Composition polymérisable conforme à la revendication 1 ou 2, **caractérisée en ce que** la charge est une charge particulaire présentant une taille moyenne de particules de 1 nm à 10 µm.

4. Composition polymérisable conforme à l'une des revendications 1 à 3, **caractérisée en ce que** la charge est une charge en nanoparticules de taille homogène, à base de silice, d'oxydes des éléments zirconium, titane, aluminium, yttrium, lanthane, cérium et/ou ytterbium, ou d'oxydes mixtes formés par ces éléments avec de la silice.

5. Composition polymérisable conforme à l'une des revendications 1 à 4, **caractérisée en ce que** la taille moyenne des particules de la charge vaut de 5 à 200 nm.

6. Composition polymérisable conforme à la revendication 5, **caractérisée en ce que** la taille moyenne des particules de la charge vaut de 10 à 100 nm.

7. Composition polymérisable conforme à la revendication 6, **caractérisée en ce que** la taille moyenne des particules de la charge vaut de 10 à 50 nm.

8. Composition polymérisable conforme à l'une des revendications 1 à 7, **caractérisée en ce que** la charge fonctionnalisée en surface avec des groupes de formule (I) porte au moins 0,01 millimole de groupes de formule (I) par gramme de charge.

9. Composition polymérisable conforme à la revendication 8, **caractérisée en ce que** la charge fonctionnalisée en surface avec des groupes de formule (I) porte de 0,05 à 2 millimoles de groupes de formule (I) par gramme de charge.

10. Composition polymérisable conforme à la revendication 9, **caractérisée en ce que** la charge fonctionnalisée en surface avec des groupes de formule (I) porte de 0,1 à 1 millimole de groupes de formule (I) par gramme de charge.

11. Composition polymérisable conforme à l'une des revendications 1 à 10, **caractérisée en ce que** la charge est en outre modifiée en surface avec au moins un autre groupe.

12. Composition polymérisable conforme à l'une des revendications 1 à 11, **caractérisée en ce qu'**elle contient au moins un monomère polymérisable par voie radicalaire.

13. Composition polymérisable conforme à l'une des revendications 1 à 12, **caractérisée en ce qu'**elle contient au moins un monomère comportant deux groupes polymérisables ou plus, et/ou au moins un monomère comportant un ou plusieurs groupe(s) acide(s).

14. Composition conforme à l'une des revendications 1 à 13, **caractérisée en ce qu'**elle contient un amorceur approprié pour une photopolymérisation

15. Composition conforme à l'une des revendications 1 à 14, **caractérisée en ce qu'**elle contient en outre une charge qui n'est pas fonctionnalisée en surface avec des groupes de formule (I).

16. Composition conforme à l'une des revendications 1 à 15, **caractérisée en ce qu'**elle contient :
a) 1 à 60 % en poids de charge fonctionnalisée en surface avec des groupes de formule (I),
b) 0 à 40 % en poids de charge non fonctionnalisée en surface avec des groupes de formule (I),
c) 1 à 70 % en poids de monomère diluant et/ou de monomère agent de réticulation,
d) 0,1 à 5,0 % en poids d'amorceur de polymérisation radicalaire,
e) et 0 à 70 % en poids de monomère acide et/ou de solvant.

17. Procédé de préparation d'une composition conforme à l'une des revendications 1 à 16, dans lequel on fait réagir la charge avec au moins un silane, et l'on mélange la charge fonctionnalisée en surface ainsi obtenue avec les autres constituants de la composition.

18. Procédé conforme à la revendication 17, dans lequel le silane présente la formule (II) :
(A)ₐ-Z-Y-R²-SiXₙR¹₃₋ₙ (II)
dans laquelle
- X représente un atome d'halogène, un groupe hydroxyle, ou un groupe alcoxy en C₁₋₅ ou acyloxy en C₁₋₃,
- l'indice n vaut de 1 à 3,
- et les symboles R¹, R², Y, Z et A ont les significations indiquées dans l'une des revendications 1 à 16.

19. Procédé conforme à la revendication 17 ou 18, dans lequel
a) on mélange le silane sous forme liquide avec la charge,
b) et l'on fait sécher la charge pour séparer les produits de condensation.

20. Procédé conforme à la revendication 17 ou 18, dans lequel
a) on disperse la charge dans une solution du silane dans un solvant,
b) on sépare la charge, et en option, on la lave une ou plusieurs fois avec le solvant de l'étape (a) et/ou au moins un autre solvant,
c) en option, on soumet la charge à un traitement thermique, et en option, on la lave de nouveau,
d) on fait sécher la charge,
e) et en option, on moud la charge.

21. Utilisation d'une charge fonctionnalisée en surface, dans laquelle des groupes de formule (I) :
(A)ₐ-Z-Y-R²-SiR¹₃₋ₘ-(O---)ₘ (I)
dans laquelle
- R¹ représente un groupe alkyle en C₁₋₁₅, alcényle en C₂₋₅ ou phényle,
- R² ne représente rien ou représente un groupe alcanediyle en C₁₋₆, linéaire ou ramifié,
- Y ne représente rien ou représente un groupe ou chaînon de type éther, thioéther, amide, ester ou uréthane,
- Z ne représente rien ou représente
un groupe aliphatique au moins divalent, linéaire ou ramifié, comportant de 2 à 40 atomes de carbone, qui peut être interrompu par un ou plusieurs groupes(s) ou chaînon(s) de type éther, thioéther, amide ou ester et contenir un ou plusieurs groupe(s) cycloaliphatique(s) comportant au moins trois atomes de carbone et/ou un ou plusieurs groupe(s) aromatique(s) comportant au moins six atomes de carbone,
un groupe cycloaliphatique au moins divalent, comportant au moins 3 atomes de carbone,
ou un groupe aromatique au moins divalent, comportant au moins 6 atomes de carbone,
- chaque symbole A représente indépendamment une entité de formule -COOH, -P(O)(OH)₂, -O-P(O)(OH)₂, -SO₂OH, -CHO, -C(O)-O-C(O)-, -NH-C(O)-CHO, -C(O)-CHO, -N=C=O, -C(O)-CH₂-C(O)-CH₃, ou -O-C(O)-CH₂-C(O)-CH₃,
- l'indice a vaut de 1 à 6,
- et l'indice m vaut de 1 à 3,
étant entendu :
- que les symboles R² et Z ne peuvent pas tous les deux à la fois ne rien représenter,
- que chacun des symboles R² et Z ne peut ne rien représenter que si à la fois Y aussi ne représente rien,
- et que si Z ne représente rien, l'indice a vaut 1,
sont liés à la charge par l'intermédiaire d'au moins un atome d'oxygène lié à l'atome de silicium du groupe de formule (I),
en vue de la préparation d'un matériau dentaire.

22. Utilisation conforme à la revendication 21, en vue de la préparation d'une colle ou d'un ciment.

23. Utilisation conforme à la revendication 21 ou 22, la charge étant accessible par un procédé dans lequel on fait réagir la charge avec au moins un silane.
